# EUROPÄISCHE PATENTSCHRIFT

(11) **EP 0 779 288 B1**
(45) Veröffentlichungstag und Bekanntmachung des Hinweises auf die Patenterteilung: **29.03.2000**
(21) Anmeldenummer: 96119262.2
(22) Anmeldetag: 02.12.1996
(51) Int. Cl.: C07D 311/58, C07D 405/12, A61K 31/35

(54) **Substituierte Chromanylsulfonyl(thio)harnstoffe, Verfahren zu ihrer Herstellung und ihre Verwendung in pharmazeutischen Präparaten sowie sie enthaltende pharmazeutische Präparate**
Substituted chromanylsulfonyl(thio)ureas, process for their preparation, their use in pharmaceutical compositions and preparations containing them
Chromanylsulfonyl(thio)urées, procédé pour leur préparation, leur utilisation dans des compositions pharmaceutiques et préparations les contenant

(30) Priorität: 14.12.1995 DE 19546736
(43) Veröffentlichungstag der Anmeldung: 18.06.1997
(73) Patentinhaber: HOECHST AKTIENGESELLSCHAFT, 65929 Frankfurt am Main (DE)
(72) Erfinder: Englert, Heinrich Christian, Dr., 65719 Hofheim (DE); Gerlach, Uwe, Dr., 65795 Hattersheim (DE); Mania, Dieter, Dr., 61462 Königstein (DE); Linz, Wolfgang, Dr., 55129 Mainz (DE); Gögelein, Heinz, Dr., 60259 Frankfurt (DE); Klaus, Erik, Dr., 65779 Kelkheim (DE); Crause, Peter, Dr., 63069 Offenbach (DE)

(56) Entgegenhaltungen:
- EP-A- 0 612 724
- EP-A- 0 727 416
- EP-A- 0 727 417
- DE-A- 1 518 874
- GB-A- 1 314 325
- US-A- 3 803 176

## Beschreibung

Die Erfindung betrifft substituierte Chromanylsulfonyl(thio)harnstoffe der Formel I, 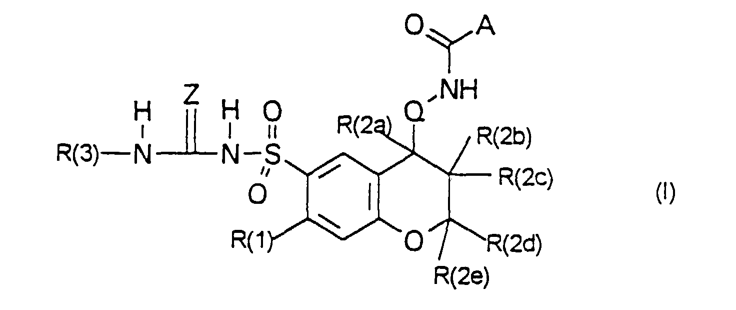 in welcher bedeuten:
- R(1): Wasserstoff, Alkyl mit 1 bis 4 C-Atomen, Alkoxy mit 1 bis 4 C-Atomen, Fluor, Chlor, Brom, lod, CF₃, NH₂, NH-Alkyl mit 1 bis 4 C-Atomen, N(Alkyl)₂ mit 1 bis 4 C-Atomen in den gleichen oder verschiedenen Alkylresten, oder S-Alkyl mit 1 bis 4 C-Atomen;
- R(2a): Wasserstoff oder Alkyl mit 1 oder 2 C-Atomen;
- R(2b) und R(2d),: die gleich oder verschieden sind, Wasserstoff, Alkyl mit 1 oder 2 C-Atomen, unsubstituiertes Phenyl, substituiertes Phenyl, unsubstituiertes Benzyl oder im Phenylrest substituiertes Benzyl, wobei als Substituenten in Phenylresten bis zu drei gleiche oder verschiedene Substituenten, ausgewählt aus der Gruppe bestehend aus Halogen, Alkyl mit 1 oder 2 C-Atomen, Alkoxy mit 1 oder 2 C-Atomen, vorhanden sind;
- R(2c) und R(2e),: die gleich oder verschieden sind, Wasserstoff oder Alkyl mit 1 oder 2 C-Atomen;
- R(3): Wasserstoff, Alkyl mit 1, 2, 3 oder 4 C-Atomen, Cycloalkyl mit 3, 4, 5 oder 6 Ring-C-Atomen, CH₂-Cycloalkyl mit 3, 4, 5 oder 6 Ring-C-Atomen, oder CF₃;
- Q: (CH₂)ₙ;
n 1 oder 2;
- Z: Schwefel oder Sauerstoff;
- A: Phenyl, das unsubstituiert ist oder durch bis zu drei gleiche oder verschiedene Substituenten, ausgewählt aus der Gruppe bestehend aus
Halogen, Alkyl mit 1 oder 2 C-Atomen, Alkoxy mit 1 oder 2 C-Atomen, substituiert ist;
oder
- A: den Rest eines gesättigten oder ungesättigten Lactams der Formel 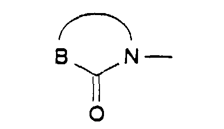
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen;
oder
- A: den Rest eines bicyclischen Systems der Formeln 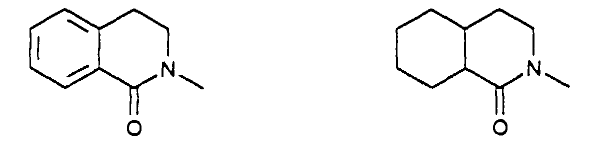 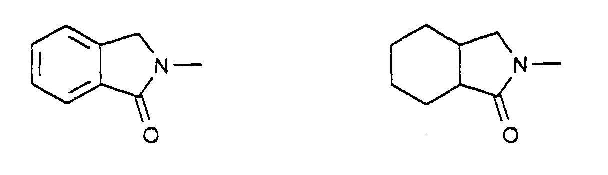
und deren physiologisch verträgliche Salze.

Bevorzugt sind Verbindungen der Formel I, in der bedeuten:
- R(1): Wasserstoff, Alkyl mit 1 oder 2 C-Atomen, Alkoxy mit 1 bis 4 C-Atomen, Fluor, Chlor, Brom, lod oder CF₃;
- R(2a), R(2b) und R(2d),: die gleich oder verschieden sind, Wasserstoff oder Alkyl mit 1 oder 2 C-Atomen;
- R(2c) und R(2e): Wasserstoff;
- R(3): Wasserstoff oder Alkyl mit 1, 2, 3 oder 4 C-Atomen;
- Q: (CH₂)ₙ;
n 1 oder 2;
- Z: Schwefel oder Sauerstoff;
- A: Phenyl, das unsubstituiert ist oder durch bis zu drei gleiche oder verschiedene Substituenten, ausgewählt aus der Gruppe bestehend aus
Halogen, Alkyl mit 1 oder 2 C-Atomen, Alkoxy mit 1 oder 2 C-Atomen, substituiert ist;
oder
- A: den Rest eines gesättigten oder ungesättigten Lactams der Formel 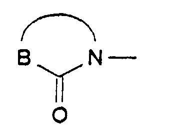
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen;
oder
- A: den Rest eines bicyclischen Systems der Formeln

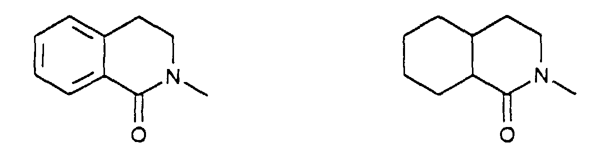

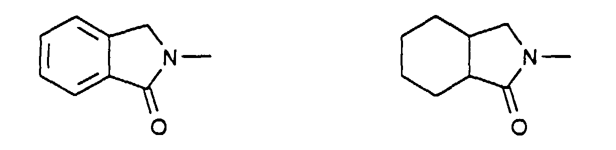

Besonders bevorzugt sind Verbindungen der Formel I, in der bedeuten:
- R(1): Wasserstoff, Alkyl mit 1 oder 2 C-Atomen, Alkoxy mit 1 oder 2 C-Atomen, Fluor, Chlor, Brom, lod oder CF₃;
- R(2a), R(2b), R(2c), R(2d), R(2e): Wasserstoff;
- R(3): Wasserstoff oder Alkyl mit 1, 2, 3 oder 4 C-Atomen;
- Q: (CH₂)ₙ;
n 1 oder 2;
- Z: Schwefel oder Sauerstoff;
- A: Phenyl, das unsubstituiert ist oder durch bis zu drei gleiche oder verschiedene Substituenten, ausgewählt aus der Gruppe bestehend aus
Halogen, Alkyl mit 1 oder 2 C-Atomen, Alkoxy mit 1 oder 2 C-Atomen, substituiert ist;
oder
- A: den Rest eines gesättigten oder ungesättigten Lactams der Formel 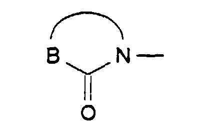
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen;
oder
- A: den Rest eines bicyclischen Systems der Formeln

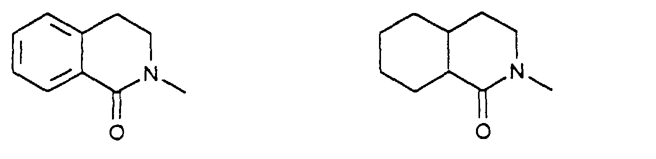

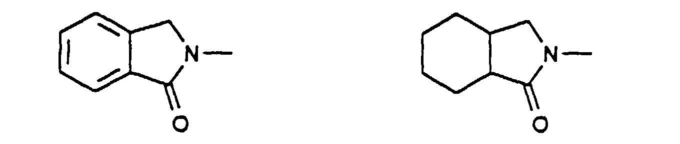

Ganz besonders bevorzugt sind Verbindungen der Formel I, in der bedeuten:
- R(1): Wasserstoff, Alkyl mit 1 oder 2 C-Atomen oder Alkoxy mit 1 oder 2 C-Atomen;
- R(2a), R(2b), R(2c), R(2d), R(2e): Wasserstoff;
- R(3): Wasserstoff oder Alkyl mit 1, 2, 3 oder 4 C-Atomen;
- Q: (CH₂)ₙ;
n 1 oder 2;
- Z: Schwefel;
- A: Phenyl, das unsubstituiert ist oder durch bis zu drei gleiche oder verschiedene Substituenten, ausgewählt aus der Gruppe bestehend aus
Halogen, Alkyl mit 1 oder 2 C-Atomen, Alkoxy mit 1 oder 2 C-Atomen, substituiert ist;
oder
- A: den Rest eines gesättigten oder ungesättigten Lactams der Formel 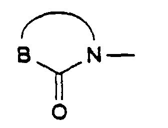
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen;
oder
- A: den Rest eines bicyclischen Systems der Formeln

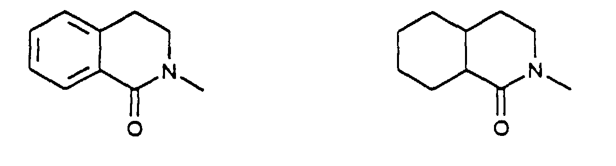

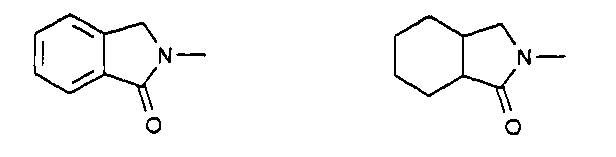

Ganz speziell bevorzugt sind Verbindungen der Formel I, in der bedeuten:
- R(1): Wasserstoff, Alkyl mit 1 oder 2 C-Atomen oder Alkoxy mit 1 oder 2 C-Atomen;
- R(2a), R(2b), R(2c), R(2d), R(2e): Wasserstoff;
- R(3): Wasserstoff, Methyl oder Ethyl;
- Q: (CH₂)ₙ;
n 1 oder 2;
- Z: Schwefel;
- A: Phenyl, das unsubstituiert oder durch bis zu drei gleiche oder verschiedene Substituenten, ausgewählt aus der Gruppe bestehend aus
Halogen, Alkyl mit 1 oder 2 C-Atomen, Alkoxy mit 1 oder 2 C-Atomen, substituiert ist;
oder
- A: den Rest eines gesättigten oder ungesättigten Lactams der Formel 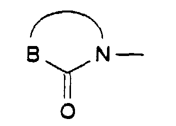
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen.

Ganz besonders speziell bevorzugt sind Verbindungen der Formel I, in der bedeuten:
- R(1): Wasserstoff, Alkyl mit 1 oder 2 C-Atomen oder Alkoxy mit 1 oder 2 C-Atomen;
- R(2a), R(2b), R(2c), R(2d), R(2e): Wasserstoff;
- R(3): Wasserstoff, Methyl oder Ethyl;
- Q: CH₂;
- Z: Schwefel;
- A: Phenyl, das unsubstituiert oder durch bis zu drei gleiche oder verschiedene Substituenten ausgewählt aus der Gruppe bestehend aus
Halogen, Alkyl mit 1 oder 2 C-Atomen, Alkoxy mit 1 oder 2 C-Atomen substituiert ist.

Ebenfalls ganz besonders bevorzugt sind auch Verbindungen der Formel I, in der bedeuten:
- R(1): Wasserstoff, Alkyl mit 1 oder 2 C-Atomen oder Alkoxy mit 1 oder 2 C-Atomen;
- R(2a), R(2b), R(2c), R(2d), R(2e): Wasserstoff;
- R(3): Wasserstoff oder Alkyl mit 1, 2, 3 oder 4 C-Atomen;
- Q: (CH₂)ₙ;
n 1 oder 2;
- Z: Sauerstoff;
- A: Phenyl, das unsubstituiert ist oder durch bis zu drei gleiche oder verschiedene Substituenten, ausgewählt aus der Gruppe bestehend aus
Halogen, Alkyl mit 1 oder 2 C-Atomen, Alkoxy mit 1 oder 2 C-Atomen, substituiert ist;
oder
- A: den Rest eines gesättigten oder ungesättigten Lactams der Formel 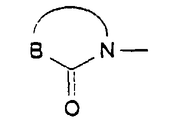
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen;
oder
- A: den Rest eines bicyclischen Systems der Formeln

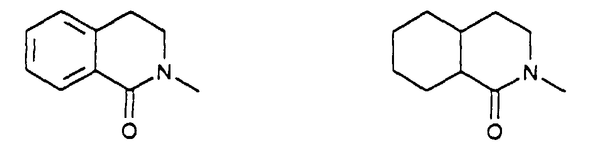

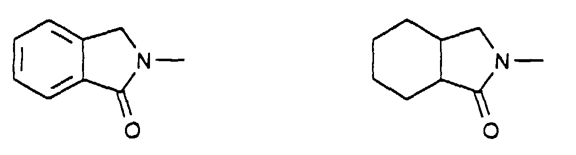

Ebenfalls ganz speziell bevorzugt sind auch Verbindungen der Formel I, in der bedeuten:
- R(1): Wasserstoff, Alkyl mit 1 oder 2 C-Atomen oder Alkoxy mit 1 oder 2 C-Atomen;
- R(2a), R(2b), R(2c), R(2d), R(2e): Wasserstoff;
- R(3): Wasserstoff, Methyl oder Ethyl;
- Q: (CH₂)ₙ;
n 1 oder 2;
- Z: Sauerstoff;
- A: Phenyl, das unsubstituiert ist oder durch bis zu drei gleiche oder verschiedene Substituenten, ausgewählt aus der Gruppe bestehend aus
Halogen, Alkyl mit 1 oder 2 C-Atomen, Alkoxy mit 1 oder 2 C-Atomen substituiert ist;
oder
- A: den Rest eines gesättigten oder ungesättigten Lactams der Formel 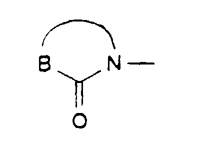
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen.

Ebenfalls ganz besonders speziell bevorzugt sind auch Verbindungen der Formel I, in der bedeuten:
- R(1): Wasserstoff, Alkyl mit 1 oder 2 C-Atomen oder Alkoxy mit 1 oder 2 C-Atomen;
- R(2a), R(2b), R(2c), R(2d), R(2e): Wasserstoff;
- R(3): Wasserstoff, Methyl oder Ethyl;
- Q: CH₂;
- Z: Sauerstoff;
- A: Phenyl, das unsubstituiert ist oder durch bis zu drei gleiche oder verschiedene Substituenten, ausgewählt aus der Gruppe bestehend aus
Halogen, Alkyl mit 1 oder 2 C-Atomen, Alkoxy mit 1 oder 2 C-Atomen, substituiert ist;
- R(2a): steht bevorzugt für Wasserstoff.

Der Begriff Alkyl bedeutet, sofern nicht anders angegeben, geradkettige oder verzweigte gesättigte Kohlenwasserstoffreste. Dies gilt auch für Alkylreste, die in Alkoxyresten enthalten sind. Beispiele für Alkylreste sind Methyl, Ethyl, n-Propyl, Isopropyl, n-Butyl, Isobutyl, sec-Butyl und tert.-Butyl. Beispiele für Cycloalkylreste sind Cyclopropyl, Cyclobutyl, Cyclopentyl und Cyclohexyl. Beispiele für Alkylen- und Alkenylenreste, die für die Gruppe B stehen, sind 1,3-Propylen, 1,4-Butylen, 1,5-Pentylen, 1,6-Hexylen, 1,3-Prop-1-enylen, 1,3-Prop-2-enylen, 1,4-But-1-enylen, 1,4-But-2-enylen, 1,4-But-3-enylen, 1,5-Pent-1-enylen, 1,5-Pent-2-enylen, 1,5-Pent-3-enylen und 1,5-Pent-4-enylen. In substituierten Phenylresten, die als solche oder in Benzylresten auftreten können, können sich die Substituenten in beliebigen Positionen befinden, bei Monosubstitution z.B. in der ortho-, meta- oder para-Position, bei Disubstitution in 2,3-, 2,4-, 2,5-, 2,6-, 3,4-, 3,5-Stellung, bei Trisubstitution z.B. in 2,3,4-, 2,3,5-, 2,3,6- oder 3,4,5-Stellung. Halogen bedeutet, sofern nicht anders angegeben, Fluor, Chlor, Brom und lod, vorzugsweise Fluor und Chlor.

Weiterhin können Verbindungen der Formel I mit Chiralitätszentren, etwa an den C-Atomen 2, 3, 4 des Chromansystems bei entsprechender Substitution, auftreten. In diesem Fall gehören alle möglichen Stereosiomeren, sowohl Enantiomere als auch Diastereomere, und auch Mischungen von zwei oder mehr Stereoisomeren in beliebigen Verhältnissen zur Erfindung, Enantiomere z.B. in enantiomerenreiner Form, wobei sowohl links- als auch rechtsdrehende Antipoden Gegenstand der Erfindung sind, als auch in Mischungen der beiden Enantiomeren in unterschiedlichen Verhältnissen.

Die Verbindungen der Formel I sind wertvolle Arzneimittelwirkstoffe für die Human- und Veterinärmedizin, insbesondere für die Behandlung von Herzrhythmusstörungen und einer verminderten Kontraktilität des Herzens. Ferner können sie als Zwischenprodukte zur Herstellung weiterer Arzneimittelwirkstoffe verwendet werden.

Für bestimmte Benzolsulfonylharnstoffe ist eine blutzuckersenkende Wirkung beschrieben. Als Prototyp solcher blutzuckersenkenden Sulfonylharnstoffe gilt das Glibenclamid, das als Mittel zur Behandlung des Diabetes mellitus therapeutisch verwendet wird und in der Forschung als vielbeachtetes Werkzeug zur Erforschung sogenannter ATP-sensitiver Kaliumkanäle dient. Neben seiner blutzuckersenkenden Wirkung besitzt das Glibenclamid noch andere Wirkungen, die bislang therapeutisch noch nicht eingesetzt werden können, die aber allesamt auf Blockade eben dieser ATP-sensitiven Kalium-Kanäle zurückgeführt werden. Dazu gehört insbesondere eine antifibrillatorische Wirkung am Herzen. Bei der Behandlung des Kammerflimmerns oder seiner Vorstufen wäre jedoch eine gleichzeitige Blutzuckersenkung unerwünscht oder gar gefährlich, da sie den Zustand des Patienten weiter verschlechtern kann. Aus der Europäischen Offenlegungsschrift EP-A-612 724 sind Benzolsulfonylharnstoffe bekannt, welche Wirkungen auf das Herz-Kreislauf-System haben; ihr Effekt ist jedoch in vieler Hinsicht noch nicht befriedigend. Chromanyl-Abkömmlinge sind darin weder beschrieben noch nahegelegt.

Die EP-A-325 964 beschreibt Chroman-Verbindungen als α₂-adrenerge Antagonisten mit Wirkung gegen Depressionen, metabolische Störungen, Glaukom, Migräne und Bluthochdruck. Sie beschreibt jedoch keine Verbindungen mit einer Substitution durch Sulfonylharnstoff- oder Sulfonylthioharnstoff-Gruppierungen und legt die erfindungsgemäßen Verbindungen auch nicht nahe.

Die Erfindung betrifft weiterhin Verfahren zur Herstellung der Verbindungen der Formel I, die durch die im folgenden wiedergegebenen Reaktionsschritte gekennzeichnet sind.
(a) Chromanylsulfonylharnstoffe der Formel I, in denen R(3) eine andere Bedeutung als Wasserstoff hat und Z für Sauerstoff steht, können hergestellt werden, indem man Sulfamoylchromane der Formel II 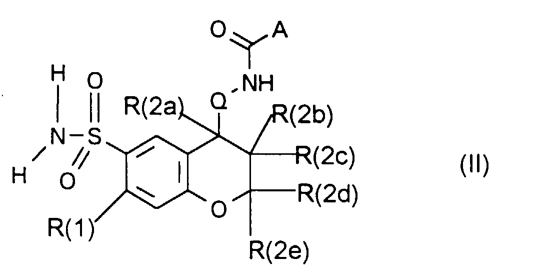 oder deren Salze der Formel III 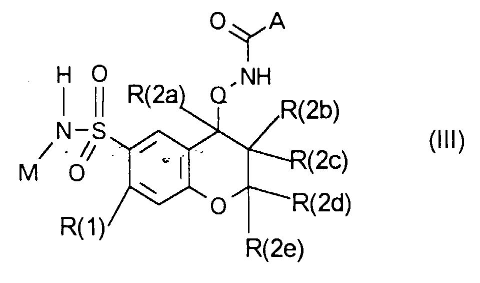 mit R(3)-substituierten Isocyanaten der Formel IV

   R(3)-N=C=O (IV)

   zu substituierten Chromanylsulfonylharnstoffen der Formel Ia (mit Z (in der Formel I) = Sauerstoff) 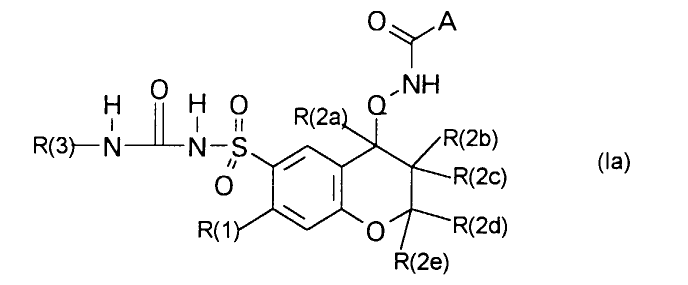 umsetzt. Die Reste in den Formeln II, III und IV haben dabei die eingangs angegebenen Bedeutungen. Als Kationen M in den Salzen der Formel III kommen dabei beispielsweise Alkali-, Erdalkali-, Ammonium- sowie Tetraalkylammoniumionen in Betracht. Äquivalent zu den R(3)-substituierten Isocyanaten der Formel IV kann man R(3)-substituierte Kohlensäurederivate wie R(3)-substituierte Carbamidsäureester, R(3)-substituierte Carbamidsäurehalogenide oder R(3)-substituierte Harnstoffe einsetzen.
(b) Chromanylsulfonylharnstoffe der Formel I, in denen R(3) für Wasserstoff und Z für Sauerstoff steht, kann man durch Umsetzung eines Sulfamoylchromans der Formel II oder von dessen Salz der Formel III mit einem Trialkylsilylisocyanat oder Siliciumtetraisocyanat und Spaltung (z. B. Hydrolyse) der primär entstehenden siliciumsubstituierten Chromanylsulfonylharnstoffe herstellen.
   Weiterhin ist es möglich, ein Sulfamoylchroman der Formel II oder dessen Salz der Formel III durch Umsetzung mit Halogencyanen und Hydrolyse der primär entstehenden N-Cyanosulfonamide mit Mineralsäuren bei Temperaturen von 0°C bis 100°C in einen Chromanylsulfonylharnstoff der Formel I umzuwandeln, in der R(3) für Wasserstoff und Z für Sauerstoff steht.
(c) Ein Chromanylsulfonylharnstoff der Formel Ia (mit Z (in der Formel I) = Sauerstoff) läßt sich aus einem Sulfamoylchroman der Formel II oder dessen Salz der Formel III mit einem an Stickstoff R(3)-substituierten Trichloracetamid der Formel V 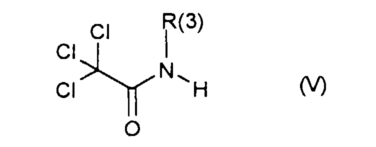 in Gegenwart einer Base in einem inerten Lösungsmittel nach Synthesis 1987, 734 - 735 bei Temperaturen von 25°C bis 150°C darstellen.
   Als Basen eignen sich zum Beispiel Alkalimetall- oder Erdalkalimetallhydroxide, -hydride, -amide oder auch -alkoholate, wie Natriumhydroxid, Kaliumhydroxid, Calciumhydroxyd, Natriumhydrid, Kaliumhydrid, Calciumhydrid, Natriumamid, Kaliumamid, Natriummethylat, Natriumethanolat, Kaliummethylat oder Kaliumethanolat. Als inerte Lösungsmittel eignen sich Ether wie Tetrahydrofuran, Dioxan, Ethylenglykoldimethylether, Diglyme, Ketone wie Aceton oder Butanon, Nitrile wie Acetonitril, Nitroverbindungen wie Nitromethan, Ester wie Ethylacetat, Amide wie Dimethylformamid (DMF) oder N-Methylpyrrolidon (NMP), Phosphorsäurehexamethyltriamid, Sulfoxide wie DMSO, Sulfone wie Sulfolan, Kohlenwasserstoffe wie Benzol, Toluol, Xylole. Weiterhin eignen sich auch Gemische dieser Lösungsmittel untereinander.
(d) Ein Chromanylsulfonylthioharnstoff der Formel Ib (mit Z (in der Formel I) = Schwefel) 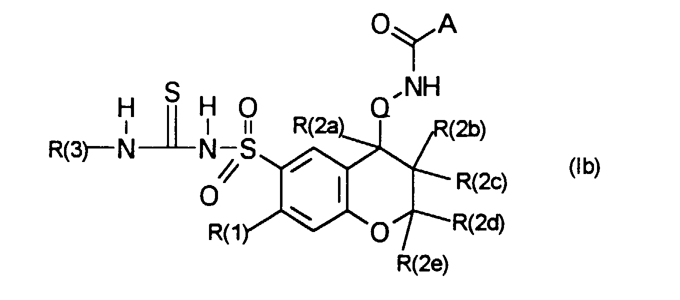 kann aus einem Sulfamoylchroman der Formel II oder dessen Salz der Formel III und einem R(3)-substituierten Isothiocyanat der Formel VI

   R(3)-N=C=S (VI)

   dargestellt werden.
(e) Einen Chromanylsulfonylthioharnstoff der Formel I, in der R(3) für Wasserstoff und Z für Schwefel steht, kann man durch Umsetzung eines Sulfamoylchromans der Formel II oder von dessen Salz der Formel III mit einem Trialkylsilylisothiocyanat, z.B. Trimethylsilylisothiocyanat, oder Siliciumtetraisothiocyanat und Spaltung (z.B. Hydrolyse) des primär entstehenden siliciumsubstituierten Chromanylsulfonylthioharnstoffs herstellen. Weiterhin ist es möglich, ein Sulfamoylchroman der Formel II oder dessen Salz der Formel III mit Benzoylisothiocyanat umzusetzen und den intermediären benzoylsubstituierten Chromanylsulfonylthioharnstoff mit einer wäßrigen Mineralsäure zur Verbindung der Formel Ib mit R(3) = H umzusetzen. Ähnliche Verfahren sind beschrieben in J. Med. Chem. 1992, 35, 1137 - 1144. Eine weitere Variante besteht darin, die unter Verfahren (b) erwähnten N-Cyansulfonamide mit Schwefelwasserstoff umzusetzen.
(f) Einen substituierten Chromanylsulfonylharnstoff der Formel Ia kann man durch eine Umwandlungsreaktion aus einem Chromanylsulfonylthioharnstoff der Formel Ib herstellen. Die Entschwefelung, also der Ersatz des Schwefelatoms im entsprechend substituierten Chromanylsulfonylthioharnstoff durch ein Sauerstoffatom, kann beispielsweise mit Hilfe von Oxiden oder Salzen von Schwermetallen oder auch durch Anwendung von Oxidationsmitteln wie Wasserstoffperoxid, Natriumperoxid oder salpetriger Säure, ausgeführt werden. Ein Thioharnstoff kann auch durch Behandlung mit Chlorierungsmitteln wie Phosgen oder Phosphorpentachlorid entschwefelt werden. Als Zwischenverbindungen werden Chlorameisensäureamidine bzw. Carbodiimide erhalten, die zum Beispiel durch Verseifen oder Anlagerung von Wasser in die entsprechenden substituierten Chromanylsulfonylharnstoffe überführt werden.
(g) Ein Chromanylsulfonylharnstoff der Formel I, in der Z für Sauerstoff steht, kann aus einem Chromanylsulfonylhalogenid z.B. der Formel VII 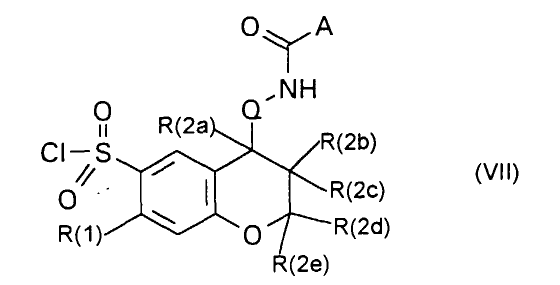 mit einem R(3)-substituierten Harnstoff oder einem R(3)-substituierten Bis(trialkylsilyl)harnstoff hergestellt werden. Weiterhin kann man das Sulfonsäurechlorid der Formel VII mit Parabansäuren zu einer Chromanylsulfonylparabansäure umsetzen, deren Hydrolyse mit Mineralsäuren den entsprechenden Chromanylsulfonylharnstoff der Formel I (Z = O) liefert.
(h) Ein Chromanylsulfonylharnstoff der Formel I, in der Z für Sauerstoff steht, läßt sich durch Umsetzung eines Amins der Formel R(3)-NH₂ mit einem Chromanylsulfonylisocyanat der Formel VIII 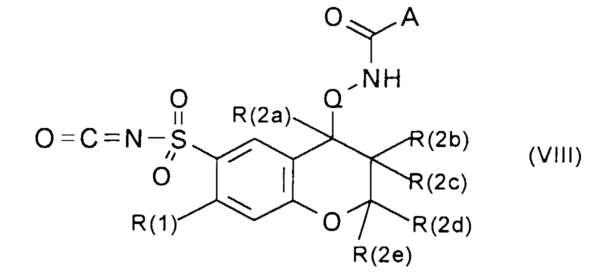 herstellen. Das Sulfonylisocyanat der Formel VIII läßt sich aus dem Sulfamoylchroman der Formel II nach üblichen Methoden, z.B. mit Phosgen, erhalten. Ebenso wie mit dem Isocyanat der Formel VIII kann ein Amin R(3)-NH₂ mit einem Chromanylsulfonylcarbamidsäureester, einem -carbamidsäurehalogenid oder einem Chromanylsulfonylharnstoff der Formel Ia, in der R(3) für Wasserstoff steht, zu einer Verbindung der Formel I, in der Z für Sauerstoff steht, umgesetzt werden.
(i) Ein Chromanylsulfonylthioharnstoff der Formel I, in der Z für Schwefel steht, läßt sich durch Umsetzen von einem Amin der Formel R(3)-NH₂ mit Chromanylsulfonylisothiocyanat der Formel IX 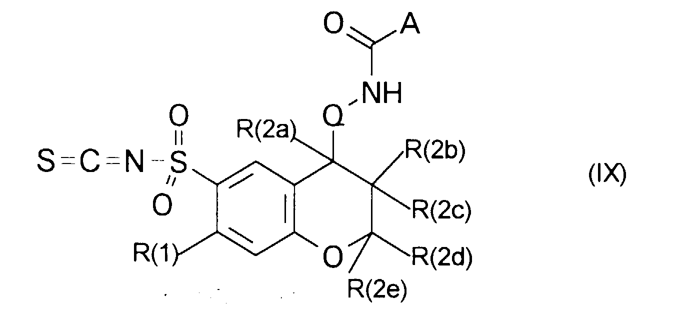 herstellen. Ebenso kann ein Amin R(3)-NH₂ mit einem Chromanylsulfonylcarbamidsäurethioester oder einem -carbamidsäurethiohalogenid zu einer Verbindung der Formel I, in der Z für Schwefel steht, umgesetzt werden.
   Die Herstellung der Sulfonylisothiocyanate der Formel IX kann durch Umsetzung eines entsprechenden Sulfonsäureamids mit Alkalihydroxid und Schwefelkohlenstoff in einem organischen Lösungsmittel, wie DMF, DMSO, N-Methylpyrrolidon, erfolgen. Das so erhaltene Di-alkalimetallsalz der Sulfonyldithiocarbaminsäure kann in einem inertem Lösungsmittel mit einem leichten Überschuß von Phosgen bzw. von einem Phosgenersatzstoff wie Triphosgen, mit einem Chlorameisensäureester (2 Äquivalente) oder mit Thionylchlorid umgesetzt werden. Die so erhaltene Lösung des Sulfonylisothiocyanats kann direkt mit den entsprechenden Aminen oder Ammoniak umgesetzt werden.
(j) Ein entsprechend substituierter Chromanylsulfenyl- oder -sulfinylharnstoff läßt sich mit einem Oxidationsmittel, wie Wasserstoffperoxid, Natriumperoxid oder salpetriger Säure, zum Chromanylsulfonylharnstoff der Formel I, in der Z für Sauerstoff steht, oxidieren.

Die Ausgangsverbindungen für die erwähnten Syntheseverfahren der Chromanylsulfonylharnstoffe der Formel I werden nach an sich bekannten Methoden hergestellt, wie sie in der Literatur (zum Beispiel in den Standardwerken wie Houben-Weyl, Methoden der Organischen Chemie, Georg Thieme Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Ausgangsstoffe können gewünschtenfalls auch in situ gebildet werden, derart, daß man sie aus dem Reaktionsgemisch nicht isoliert, sondern sofort weiter umsetzt.

4-Aminoalkylchromane sind beispielsweise in der europäischen Offenlegungsschrift EP-A-325 964 und den US-Patenten 5 140 039 oder 5 185 364 beschrieben oder können nach den dort angegebenen Methoden hergestellt werden. Geeignet substituierte Amine der Formel XI kann man wie in Schema 1 angegeben zu den Amiden der Formel XII acylieren und dann einer Halogensulfonierung unterwerfen. Als acylierende Mittel für Aminogruppen eignen sich zweckmäßig die Alkylester, Halogenide (zum Beispiel Chloride oder Bromide) oder Anhydride von Carbonsäuren der Formel

R(5)COY.

R(5) steht hierbei für einen Trihalogenmethylrest oder einen (C₁-C₄)-Alkylrest, oder R(5)COY steht für ein Benzoesäurederivat der Formel ACOY, wobei A hier gemäß der eingangs erwähnten Bedeutung dieses Restes für substituiertes oder unsubstituiertes Phenyl steht. Y ist eine Fluchtgruppe wie Halogenid, (C₁-C₄)-Alkoxy, Trihalogenacetat, (C₁-C₄)-Carboxylat.

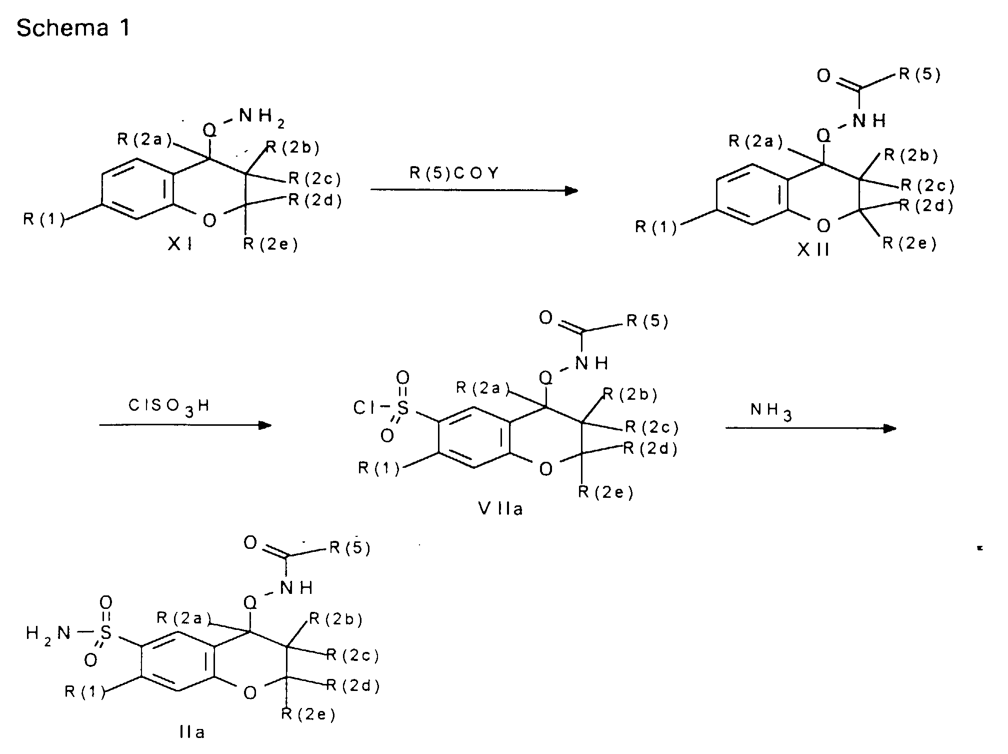

Die Synthesen der Verbindungen der Formel XII werden üblicherweise unter Zusatz einer tertiären Base wie zum Beispiel Pyridin oder einem Trialkylamin in Gegenwart oder Abwesenheit eines inerten Lösungsmittels durchgeführt, wobei auch ein Katalysator wie zum Beispiel Dimethylaminopyridin zugegen sein kann. Die Umsetzung kann bei Temperaturen von etwa 0°C bis 160°C, vorzugsweise von 20 bis 150°C erreicht werden. Die Acylgruppe der Verbindungen der Formel XII kann sowohl eine Schutzgruppe sein, als auch, im Fall der Benzoesäurederivate, d.h. wenn R(5) für A mit der oben erläuterten Bedeutung von A steht, Teil der Verbindung der Formel I sein. Als inerte Lösungsmittel eignen sich Ether wie Tetrahydrofuran, Dioxan, Glykolether wie Ethylenglykolmonomethyl- oder monoethylether (Methylglykol oder Ethylglykol), Ethylenglykoldimethylether, Diglyme, Ketone wie Aceton oder Butanon, Nitrile wie Acetonitril, Amide wie Dimethylformamid (DMF) oder N-Methylpyrrolidon (NMP), Phosphorsäurehexamethyltriamid, Sulfoxide wie DMSO, chlorierte Kohlenwasserstoffe wie Dichlormethan, Chloroform, Trichlorethylen, 1,2-Dichlorethan oder Kohlenstofftetrachlorid, Kohlenwasserstoffe wie Benzol, Toluol, Xylole. Weiterhin eignen sich auch Gemische dieser Lösungsmittel untereinander.

Steht in den Verbindungen der Formel I die Gruppe A für den Rest der Formel 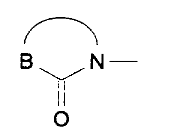 mit der eingangs festgelegten Definition, von B, oder steht B für einen Rest der Formeln 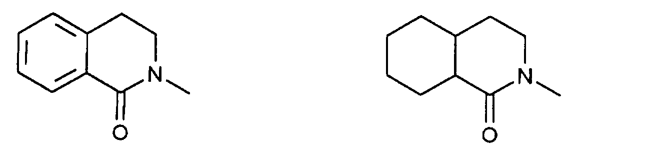 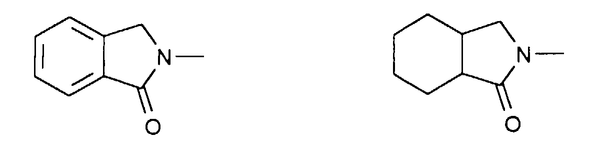 dann können die der Formel XII entsprechenden acylierten Amine wie folgt hergestellt werden:
Das Amin der Formel XI wird zunächst in ein Isocyanat oder ein reaktives Kohlensäurederivat überführt. Die Umwandlung des Amins XI in ein Isocyanat (Schema 2) kann in bekannter Weise durch Reaktion von XI mit

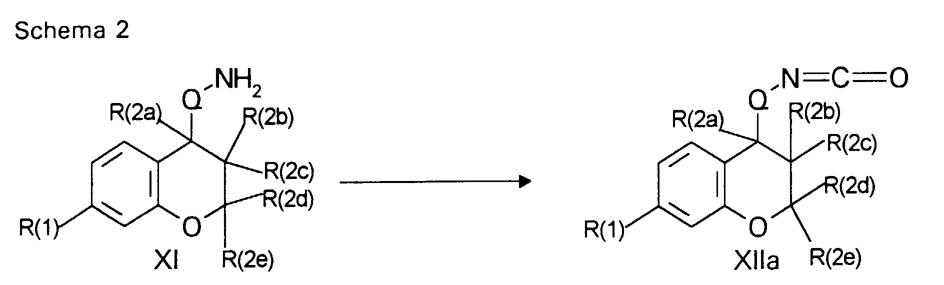

Kohlensäurehalogeniden wie Phosgen oder Triphosgen in Gegenwart tertiärer Alkylamine oder Pyridin und inerter Solventien erfolgen. Als inerte Lösungsmittel eignen sich Ether wie Tetrahydrofuran, Dioxan, Ethylenglykoldimethylether, Diglyme, Ketone wie Aceton oder Butanon, Nitrile wie Acetonitril, Nitroverbindungen wie Nitromethan, Ester wie Ethylacetat, Amide wie Dimethylformamid (DMF) oder N-Methylpyrrolidon (NMP), Phosphorsäurehexamethyltriamid, Sulfoxide wie DMSO, Sulfone wie Sulfolan, Kohlenwasserstoffe wie Benzol, Toluol, Xylole. Weiterhin eignen sich auch Gemische dieser Lösungsmittel untereinander. Als reaktive Kohlensäurederivate kommen Kohlensäureester in Betracht, wie sie sich aus Chlorameisensäurealkylestern und XI sowie geeigneten tertiären Alkylaminen oder Pyridin synthetisieren lassen. Weiterhin können N,N'-Carbonyldiimidazol und analoge reaktive Derivate als Isocyanatäquivalente eingesetzt werden (Staab, H.A., Synthesen mit heterocyclischen Amiden (Azoliden), Angewandte Chemie 74 (1962), Nr. 12, S. 407-423).

Das Isocyanat der Formel XII a oder entsprechende Urethane können dann zur Einführung der zweiten Molekülkomponente mit einer Verbindung der Formel 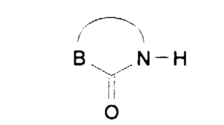 mit der eingangs erwähnten Bedeutung von B oder einer Verbindung der Formel 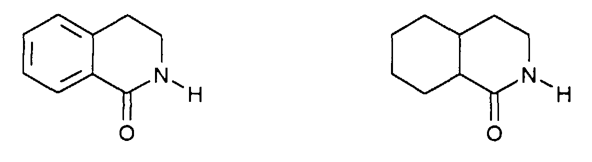 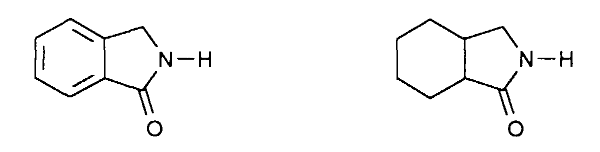 in An- oder Abwesenheit inerter Lösungsmittel bei Temperaturen von z.B. 100 - 170°C gekoppelt werden (Justus Liebigs Ann. Chem. 1956, 598, S. 203) und liefern die der Formel XII entsprechenden Acylharnstoff-Derivate der Formel XIIb, in der A für die eingangs erwähnten heterocyclischen Reste steht (Schema 3).

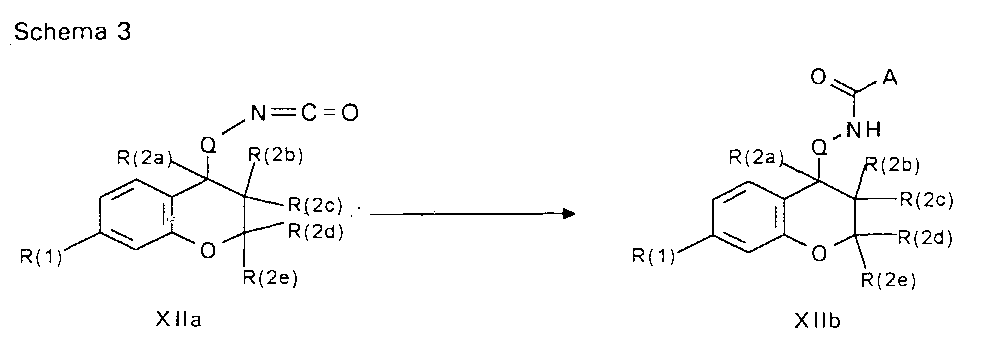

Die gemäß Schema 1 oder 2/3 erhaltenen acylierten Amine der Formeln XII bzw. XIIb können in bekannter Weise in die Sulfonamide der Formel II überführt werden. Die Sulfonamide der Formel II werden nach an sich bekannten Methoden hergestellt und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Synthesen können gewünschtenfalls in einem, zwei oder mehreren Schritten vollzogen werden. Insbesondere sind Verfahren bevorzugt, in denen das acylierte Amin der Formel XII bzw. XIIb durch elektrophile Reagenzien in An- oder Abwesenheit von inerten Lösungsmitteln bei Temperaturen von -10°C bis 120°C, vorzugsweise von 0°C bis 100°C, in aromatische Sulfonsäuren oder deren Derivate, wie zum Beispiel Sulfonsäurehalogenide, überführt wird. Beispielsweise können Sulfonierungen mit Schwefelsäuren oder Oleum oder Halogensulfonierungen mit Halogensulfonsäuren, Reaktion mit Sulfurylhalogeniden in Gegenwart von wasserfreien Metallhalogeniden oder mit Thionylhalogeniden in Gegenwart von wasserfreien Metallhalogeniden mit anschließender, in bekannter Weise durchgeführter Oxidation zu Sulfonsäurechloriden, durchgeführt werden. Sind Sulfonsäuren die primären Reaktionsprodukte, so können diese entweder direkt oder durch Behandlung mit tertiären Aminen, wie zum Beispiel Pyridin oder Trialkylaminen, oder mit Alkali- oder Erdalkalihydroxiden oder Reagenzien, die diese basischen Verbindungen in situ bilden, in bekannter Weise durch Säurehalogenide wie zum Beispiel Phosphortrihalogenide, Phosphorpentahalogenide, Phosphoroxychloride, Thionylhalogenide, Oxalylhalogenide, in Sulfonsäurehalogenide überführt werden. Die Überführung der Sulfonsäurederivate in Sulfonamide erfolgt in literaturbekannter Weise, vorzugsweise werden Sulfonsäurechloride in inerten Lösungsmitteln bei Temperaturen von 0°C bis 100°C mit wäßrigem Ammoniak umgesetzt. Weiterhin kann man Sulfonamide nach literaturbeschriebenen Verfahren aus den nach Schema 1 hergestellten acylierten Aminen der Formel XII durch Umsetzungen mit alkali- oder erdalkalimetallorganischen Reagenzien in inerten Lösungsmitteln und unter Inertgasatmosphäre bei Temperaturen von -100°C bis 50°C, vorzugsweise von -100°C bis 30°C, Umsetzung mit Schwefeldioxid und anschließende thermische Behandlung mit Amidosulfonsäure synthetisieren.

Fungiert die Acylgruppe R(5)CO als Schutzgruppe für die Aminogruppe in der Verbindung der Formel XI, dann läßt sich diese nach Darstellung des Sulfonamids der Formel IIa mit Säuren oder Basen abspalten. Durch Spaltung mit wäßrigen Säuren oder Säuren in inerten Lösungsmitteln kann das zugehörige Säureadditionssalz entstehen. Für diese Umsetzung kommen zum Beispiel Schwefelsäure, Halogenwasserstoffsäuren, wie Chlorwasserstoffsäure oder Bromwasserstoffsäure, Phosphorsäuren wie Orthophosphorsäure oder Polyphosphorsäure oder andere übliche Säuren in Betracht, mit denen Amide gespalten werden können. Die Spaltung des acylierten Amins der Formel XII mit Basen kann auch in wäßrigen oder inerten Lösungsmitteln erfolgen. Als Basen eignen sich zum Beispiel Alkalimetall- oder Erdalkalimetallhydroxide oder auch -alkoholate, wie Natriumhydroxid, Kaliumhydroxid, Calciumhydroxyd, Natriumhydrid, Natriummethylat, Natriumethanolat, Kaliummethylat oder Kaliumethanolat.

Aus den so hergestellten sulfonamidsubstituierten Aminen oder deren Säureadditionsverbindungen können wie oben erwähnt die Sulfamoylchromane der Formel II hergestellt werden, in denen das Stickstoffatom die Acylgruppe ACO trägt. Je nach der Natur der Glieder R(1), R(2a), R(2b), R(2c), R(2d), R(2e), R(3), Z, Q, und A wird in einzelnen Fällen das eine oder andere der genannten Verfahren für die Herstellung der Verbindungen der Formel I ungeeignet sein oder zumindest Vorkehrungen zum Schutz reaktiver Gruppen notwendig machen. Derartige, verhältnismäßig selten auftretende Fälle können vom Fachmann unschwer erkannt werden, und es bereitet keine Schwierigkeiten, in solchen Fällen einen anderen der beschriebenen Synthesewege erfolgreich anzuwenden.

Die Verbindungen der Formel I können ein oder mehrere chirale Zentren besitzen. Liegen ein oder mehrere chirale Zentren vor, so sind Verbindungen der Formel I mit einheitlicher Stereochemie an diesen Zentren bevorzugt. Verbindungen der Formel I mit einem oder mehreren chiralen Zentren können bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Isomeren, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen eignen sich zum Beispiel optisch aktive Säuren, wie die R- bzw. R,R- und S- bzw. S,S-Formen von Weinsäure, Dibenzoylweinsäure, Diacetylweinsäure, Camphersulfonsäure, Mandelsäure, Äpfelsäure oder Milchsäure. Die verschiedenen Formen der Diastereomeren können in an sich bekannter Weise, zum Beispiel durch fraktionierte Kristallisation, getrennt und die Enantiomeren der Formel I können in an sich bekannter Weise aus den Diastereomeren in Freiheit gesetzt werden. Enantiomerentrennungen gelingen ferner durch Chromatographie an optisch aktiven Trägermaterialien. Ein besonders einfaches Verfahren zur Herstellung von optisch einheitlichen Verbindungen besteht beispielsweise darin, daß man die Amine der Formel XI durch Umkristallisation mit optisch aktiven Säuren wie beispielsweise (+)- oder (-)-Mandelsäure in die Enantiomeren spaltet und dann wie oben beschrieben in die Endverbindungen der Formel I umwandelt, die nun ihrerseits enantiomerenrein sind.

Die Verbindungen der Formel I sowie deren physiologisch unbedenkliche Salze sind wertvolle Therapeutika, die sich nicht nur als Antiarrhythmika, sondern auch zur Behandlung und Prophylaxe bei Störungen des cardiovaskulären Systems, Herzinsuffizienz, Herztransplantationen oder cerebralen Gefäßerkrankungen an Menschen oder Säugetieren (zum Beispiel Affen, Hunde, Mäuse, Ratten, Kaninchen, Meerschweinchen, Katzen und größere Nutztiere, z.B. Rinder und Schweine) eignen. Unter physiologisch unbedenklichen Salzen der Verbindungen der Formel I versteht man nach Remmington's Pharmaceutical Science, 17. Auflage, 1985, Seiten 14 - 18, beispielsweise Verbindungen der Formel X, 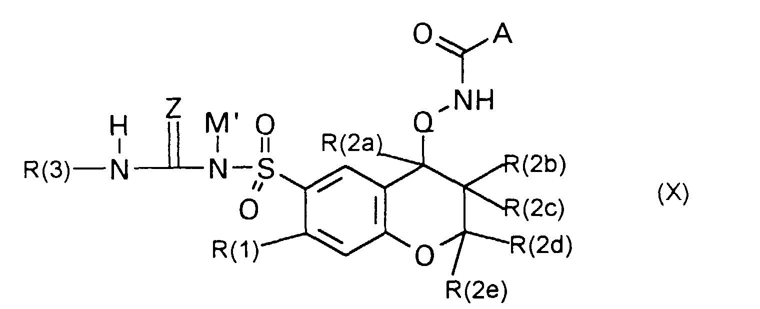 die sich aus nicht toxischen organischen und anorganischen Basen und Chromanylsulfonyl(thio)harnstoffen der Formel I darstellen lassen. Bevorzugt sind hierbei Salze, in denen das Kation M' in der Formel X ein Natrium-, Kalium-, Rubidium-, Calcium-, Magnesium-, Ammoniumion oder ein Ammoniumion mit organischen Resten ist, sowie die Säureadditionsprodukte aus Verbindungen der Formel I und basischen Aminosäuren, wie zum Beispiel Lysin oder Arginin. Die Salze können gemäß der üblichen Vorgehensweise beispielsweise durch Umsetzung der Verbindungen der Formel I mit geeigneten Basen, wie z.B. Natrium- oder Kaliumhydroxid oder einem Amin, in einem Lösungs- oder Verdünnungsmittel erhalten werden. Als physiologisch unbedenkliche Salze kommen bei Verbindungen der Formel I mit basischen Gruppen weiterhin die Additionsprodukte mit nicht toxischen anorganischen und organischen Säuren in Betracht, die ebenfalls beispielsweise durch Vereinigen der Komponenten in einem geeigneten Lösungs- oder Verdünnungsmittel erhalten werden können. Geeignete Säuren sind zum Beispiel Schwefelsäure, Halogenwasserstoffsäuren, wie Chlorwasserstoffsäure oder Bromwasserstoffsäure, Phosphorsäuren wie Orthophosphorsäure, Polyphosphorsäure, Sulfaminsäure, ferner organische Säuren, insbesondere aliphatische, alicyclische, araliphatische, aromatische oder heterocyclische einoder mehrbasige Carbon-, Sulfon- oder Schwefelsäuren, zum Beispiel Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Phenylessigsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und -disulfonsäuren, Laurylschwefelsäure.

Die Verbindungen der vorliegenden Erfindung sind insbesondere wertvolle Arzneimittel zur Behandlung von Herzrhythmusstörungen unterschiedlichster Genese und zur Verhinderung des arrhythmisch bedingten, plötzlichen Herztodes und können daher als Antiarrhythmika Verwendung finden. Beispiele arrhythmischer Störungen des Herzens sind supraventrikuläre Rhythmusstörungen wie etwa Vorhof-Tachycardien, Vorhof-Flattern oder paroxysmale supraventriculäre Rhythmusstörungen, oder ventrikuläre Rhythmusstörungen wie ventrikuläre Extrasystolen, insbesondere aber lebensbedrohende ventrikuläre Tachycardien oder das besonders gefährliche Kammerflimmern. Sie eignen sich insbesondere für solche Fälle, wo Arrhythmien Folge einer Verengung eines Koronargefäßes sind, wie sie beispielsweise bei Angina Pectoris oder während eines akuten Herzinfarkts oder als chronische Folge eines Herzinfarkts auftreten. Sie sind daher insbesondere bei Postinfarktpatienten zur Verhinderung des plötzlichen Herztodes geeignet.

Weitere Krankheitsbilder, wo derartige Rhythmusstörungen und/oder der plötzliche, arrhythmisch bedingte Herztod eine Rolle spielen, sind beispielsweise die Herzinsuffizienz oder die Herzhypertrophie als Folge eines chronisch erhöhten Blutdrucks.

Darüberhinaus sind die Verbindungen der vorliegenden Erfindung in der Lage, eine verminderte Kontraktilität des Herzens positiv zu beeinflussen. Hierbei kann es sich um ein krankheitsbedingtes Nachlassen der Herzkontraktilität handeln, wie beispielsweise bei Herzinsuffizienz, aber auch um akute Fälle wie Herzversagen bei Schockeinwirkungen. Ebenso kann bei einer Herztransplantation das Herz nach erfolgter Operation seine Leistungsfähigkeit rascher und zuverlässiger wieder aufnehmen. Gleiches gilt für Operationen am Herz, die eine vorübergehende Still-Legung der Herzaktivität durch cardioplegische Lösungen erforderlich machen.

Die erfindungsgemäßen Verbindungen der Formel I und ihre physiologisch unbedenklichen Salze können zur Herstellung pharmazeutischer Zubereitungen verwendet werden. Hierbei können sie zusammen mit mindestens einem festen oder flüssigen Träger oder Hilfsstoff allein oder in Kombination mit anderen Arzneimitteln, z.B. Herz-Kreislauf-aktiven Arzneimitteln wie etwa Calcium-Antagonisten oder ACE-Hemmern, in eine geeignete Dosierungsform gebracht werden. Pharmazeutische Zubereitungen und Arzneimittel, die eine wirksame Menge einer oder mehrerer Verbindungen der Formel I oder ihrer physiologisch unbedenklichen Salze enthalten, die Verwendung der Verbindungen zur Herstellung von Arzneimitteln und Verfahren zur Herstellung solcher Arzneimittel sind ebenfalls Gegenstand der vorliegenden Erfindung. Diese Zubereitungen können als Arzneimittel in der Human- oder Veterinärmedizin verwendet werden.

Als Trägerstoffe kommen organische oder anorganische Substanzen in Frage, die sich für die enterale (zum Beispiel die orale) oder parenterale (zum Beispiel die intravenöse) Applikation oder für topische Anwendungen eignen und mit den Verbindungen der Formel I nicht reagieren, beispielsweise Wasser, pflanzliche Öle, Alkohole wie Ethanol, Propandiol oder Benzylalkohole, Polyethylenglykole, Polypropylenglykole, Glycerintriacetat, Gelatine, Kohlenhydrate, wie Lactose oder Stärke, Magnesiumstearat, Talk, Lanolin, Vaseline. Zur oralen und rektalen Anwendung dienen insbesondere Arzneiformen wie Tabletten, Dragees, Kapseln, Suppositorien, Lösungen, vorzugsweise ölige oder wäßrige Lösungen, Sirupe, Säfte oder Tropfen, ferner Suspensionen oder Emulsionen, für die topische Anwendung Salben, Cremes, Pasten, Lotionen, Gele, Sprays, Schäume, Aerosole, Lösungen (zum Beispiel in Alkoholen, wie Ethanol, Isopropanol oder 1,2-Propandiol oder deren Gemische untereinander oder mit Wasser) oder Puder. Als weitere Arzneiformen kommen z.B. auch Implantate in Betracht. Die Verbindungen der Formel I können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht. Die pharmazeutischen Zubereitungen können Hilfsstoffe wie Gleit-, Konservierungs-, Stabilisierungs- und/oder Netzmittel, Emulgatoren, Salze (z.B. zur Beeinflussung des osmotischen Druckes), Puffersubstanzen, Farb- und Geschmacks- und/ oder Aromastoffe enthalten. Sie können, falls erwünscht, auch einen oder mehrere weitere Wirkstoffe enthalten, zum Beispiel ein oder mehrere Vitamine.

Die Dosierungen, die zur Behandlung von Herzrhythmusstörungen mit den Verbindungen der Formel I notwendig sind, hängen davon ab, ob akut oder prophylaktisch therapiert wird, und richten sich nach dem jeweiligen Einzelfall. Normalerweise kommt man mit einem Dosisbereich von etwa mindestens 0.01, vorzugsweise 0.1 mg, insbesondere 1 mg bis höchstens 100 mg, vorzugsweise 10 mg pro kg und Tag aus, wenn Prophylaxe betrieben wird. Besonders geeignet ist ein Dosisbereich von 1 bis 10 mg pro kg und Tag. Die Dosis kann dabei in Form einer oralen oder parenteralen Einzeldosis verabreicht werden oder in mehrere, insbesondere z.B. bis zu vier Einzeldosen aufgeteilt werden. Werden akute Fälle von Herzrhythmusstörungen behandelt, beispielsweise auf einer Intensivstation, kann die parenterale Verabreichung z.B. durch Injektion oder Infusion, vorteilhaft sein. Ein bevorzugter Dosisbereich in kritischen Situationen kann dann 10 bis 100 mg betragen und beispielsweise als intravenöse Dauerinfusion verabreicht werden.

Erfindungsgemäß können außer den in den Ausführungsbeispielen beschriebenen Verbindungen beispielsweise auch die in der folgenden Tabelle zusammengestellten Verbindungen der Formel I erhalten werden:
4-(5-Chlor-2-methoxy-benzamidomethyl)-6-(methylaminocarbonylaminosulfonyl)-7-ethylchroman
4-(5-Brom-2-methoxy-benzamidomethyl)-6-(methylaminocarbonylaminosulfonyl)-7-ethylchroman
4-(5-Brom-2-methoxy-benzamidomethyl)-6-(methylaminothiocarbonylaminosulfonyl)-7-methoxychroman
4-(5-Chlor-2-methoxy-benzamidoethyl)-6-(methylaminocarbonylaminosulfonyl)-7-ethylchroman
4-(5-Chlor-2-methoxy-benzamidoethyl)-6-(methylaminothiocarbonylaminosulfonyl)-7-methoxychroman
4-(5-Brom-2-methoxy-benzamidoethyl)-6-(methylaminocarbonylaminosulfonyl)-7-ethylchroman
4-(5-Brom-2-methoxy-benzamidoethyl)-6-(methylaminothiocarbonylaminosulfonyl)-7-mEthoxychroman
4-(5-Chlor-2-methoxy-benzamidoethyl)-6-(methylaminocarbonylaminosulfonyl)-7-methoxychroman
4-(5-Chlor-2-methoxy-benzamidoethyl)-6-(methylaminothiocarbonylaminosulfonyl)-7-ethoxychroman
4-(5-Chlor-2-methoxy-benzamidomethyl)-6-(ethylaminocarbonylaminosulfonyl)-7-ethylchroman
4-(5-Brom-2-methoxy-benzamidomethyl)-6-(ethylaminocarbonylaminosulfonyl)-7-ethylchroman
4-(5-Brom-2-methoxy-benzamidomethyl)-6-(ethylaminothiocarbonylaminosulfonyl)-7-methoxychroman
4-(5-Chlor-2-methoxy-benzamidoethyl)-6-(ethylaminocarbonylaminosulfonyl)-7-ethylchroman
4-(5-Chlor-2-methoxy-benzamidoethyl)-6-(ethylaminothiocarbonylaminosulfonyl)-7-methoxychroman
4-(5-Brom-2-methoxy-benzamidoethyl)-6-(ethylaminocarbonylaminosulfonyl)-7-ethylchroman
4-(5-Brom-2-methoxy-benzamidoethyl)-6-(ethylaminothiocarbonylaminosulfonyl)-7-methoxychroman
4-(5-Chlor-2-methoxy-benzamidoethyl)-6-(ethylaminocarbonylaminosulfonyl)-7-methoxychroman
4-(5-Chlor-2-methoxy-benzamidoethyl)-6-(ethylaminothiocarbonylaminosulfonyl)-7-ethoxychroman
4-(5-Fluor-2-methoxy-benzamidomethyl)-6-(ethylaminocarbonylaminosulfonyl)-7-methoxychroman
4-(5-Fluor-2-methoxy-benzamidomethyl)-6-(methylaminocarbonylaminosulfonyl)-7-methylchroman
4-(5-Fluor-2-methoxy-benzamidomethyl)-6-(methylaminothiocarbonylaminosulfonyl)-7-methylchroman
4-(5-Fluor-2-methoxy-benzamidomethyl)-6-(methylaminocarbonylaminosulfonyl)-7-ethylchroman
4-(5-Fluor-2-methoxy-benzamidomethyl)-6-(methylaminothiocarbonylaminosulfonyl)-7-ethylchroman
4-(5-Fluor-2-methoxy-benzamidomethyl)-6-(methylaminocarbonylaminosulfonyl)-7-ethoxychroman
4-(5-Fluor-2-methoxy-benzamidomethyl)-6-(methylaminothiocarbonylaminosulfonyl)-7-ethoxychroman
4-(5-Fluor-2-methoxy-benzamidomethyl)-6-(ethylaminocarbonylaminosulfonyl)-7-ethoxychroman
4-(5-Fluor-2-methoxy-benzamidomethyl)-6-(ethylaminothiocarbonylaminosulfonyl)-7-ethoxychroman
4-((3-Ethyl-4-methyl-2-oxo-3-pyrrolin-1-carboxamido)ethyl)-6-(methylaminothiocarbonylaminosulfonyl)-7-ethoxychroman
4-((3-Ethyl-4-methyl-2-oxo-3-pyrrolin-1-carboxamido)ethyl)-6-(methylaminocarbonylaminosulfonyl)-7-ethoxychroman
4-((3-Ethyl-4-methyl-2-oxo-3-pyrrolin-1-carboxamido)ethyl)-6-(methylaminothiocarbonylaminosulfonyl)-7-ethylchroman
4-((3-Ethyl-4-methyl-2-oxo-3-pyrrolin-1-carboxamido)ethyl)-6-(methylaminocarbonylaminosulfonyl)-7-ethylchroman
4-((2-Oxo-3-pyrrolin-1-carboxamido)ethyl)-6-(methylaminothiocarbonylaminosulfonyl)-7-ethoxychroman
4-((2-Oxo-3-pyrrolin-1-carboxamido)ethyl)-6-(methylaminocarbonylaminosulfonyl)-7-ethoxychroman
4-((2-Oxo-3-pyrrolin-1-carboxamido)ethyl)-6-(methylaminothiocarbonylaminosulfonyl)-7-ethylchroman
4-((2-Oxo-3-pyrrolin-1-carboxamido)ethyl)-6-(methylaminocarbonylaminosulfonyl)-7-ethylchroman
4-((2-Oxo-3-pyrrolin-1-carboxamido)ethyl)-6-(methylaminothiocarbonylaminosulfonyl)-7-ethoxychroman
4-((2-Oxo-3-pyrrolin-1-carboxamido)ethyl)-6-(methylaminocarbonylaminosulfonyl)-7-ethoxychroman

### Beispiel 1:

### 4-(5-Chlor-2-methoxy-benzamidomethyl)-6-(methylaminocarbonylaminosulfonyl)-7-methoxychroman

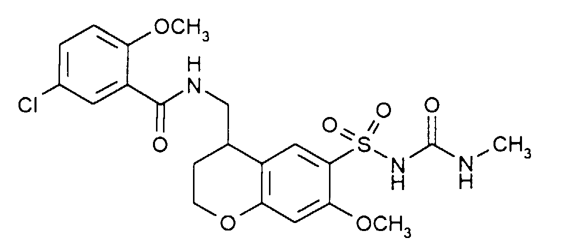

1.76 g (4 mmol) 4-(5-Chlor-2-methoxy-benzamidomethyl)-6-sulfamoyl-7-methoxychroman werden in 10 ml trockenem DMSO gelöst und nach Zugabe von 0.4 g (10 mmol) pulverisiertem Natriumhydroxid und 1.05 g (6 mmol) N-Methyltrichloracetamid 30 Minuten bei 80°C erhitzt. Der kühle Reaktionsansatz wird in Eiswasser eingetragen, mit Aktivkohle geklärt und auf pH 1 angesäuert. Der Niederschlag wird abgesaugt, getrocknet und zweimal aus Ethanol/DMF umkristallisiert. 4-(5-Chlor-2-methoxy-benzamidomethyl)-6-(methylaminocarbonylaminosulfonyl)-7-methoxychroman schmilzt bei 207°C.

### Herstellung der Ausgangsverbindung 4-(5-Chlor-2-methoxy-benzamidomethyl)-6-sulfamoyl-7-methoxychroman

14.8 g (64.4 mmol) 4-Aminomethyl-7-methoxychroman-hydrochlorid werden in 75 ml Pyridin gelöst und unter Kühlung auf 0°C mit 13.4 g 2-Methoxy-5-chlorbenzoesäurechlorid versetzt. Man rührt 1.5 Stunden bei Zimmertemperatur und 1 Stunde bei 60°C. Die abgekühlte Reaktionsmischung wird zwischen Wasser und Methylenchlorid verteilt. Die wäßrige Phase wird dreimal mit Methylenchlorid extrahiert. Die vereinigten organischen Phasen werden mit 2N Salzsäure, Wasser und Bikarbonatlösung gewaschen. Nach Trocknen und Eindampfen der organischen Phase erhält man ein Öl. 20 g dieses Öls werden auf -20°C gekühlt. Es werden 30 ml vorgekühlte Chlorsulfonsäure unter Rühren zugegeben. Unter Schütteln läßt man auf Raumtemperatur kommen und gibt weitere 5 ml Chlorsulfonsäure zu. Nach Einrühren in Eiswasser wird der erhaltene Niederschlag abgesaugt und nach Waschen mit wenig kaltem Wasser in eine auf -20°C gekühlte Lösung aus 200 ml Aceton und 120 ml konzentriertem Ammoniak eingetragen. Man läßt auf Raumtemperatur erwärmen, und nach Stehen über Nacht wird die Lösung im Vakuum eingeengt. Unter Eiskühlung wird der Rückstand mit konzentrierter Salzsäure versetzt. Der erhaltene Niederschlag wird abgesaugt und aus Eisessig/Methanol umkristallisiert. 4-(5-Chlor-2-methoxy-benzamidomethyl)-6-sulfamoyl-7-methoxychroman fällt in Form von farblosen Kristallen vom Schmelzpunkt 202°C an.

### Beispiel 2:

### 4-(5-Chlor-2-methoxy-benzamidomethyl)-6-(ethylaminocarbonylaminosulfonyl)-7-methoxychroman

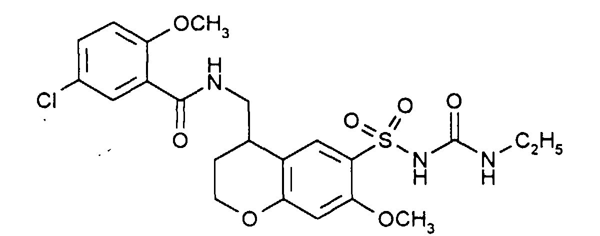

4-(5-Chlor-2-methoxy-benzamidomethyl)-6-(ethylaminocarbonylaminosulfonyl)-7-methoxychroman wird analog zu Beispiel 1 aus 4-(5-Chlor-2-methoxybenzamidomethyl)-6-sulfamoyl-7-methoxychroman und N-Ethyltrichloracetamid synthetisiert. Schmelzpunkt: 211-213°C.

### Beispiel 3:

### 4-(5-Chlor-2-methoxy-benzamidomethyl)-6-(n-propylaminocarbonylaminosulfonyl)-7-methoxychroman

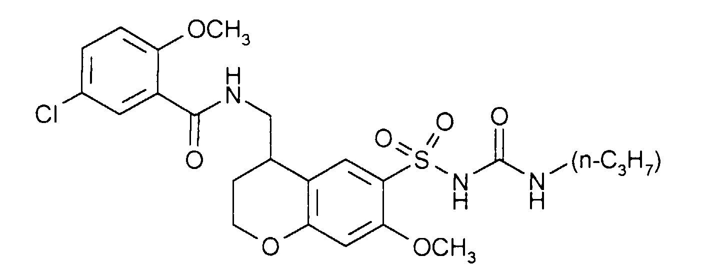

4-(5-Chlor-2-methoxy-benzamidomethyl)-6-(n-propylaminocarbonylaminosulfonyl)-7-methoxychroman wird analog zu Beispiel 1 aus 4-(5-Chlor-2-methoxy-benzamidomethyl)-6-sulfamoyl-7-methoxychroman und N-(n-Propyl)trichloracetamid hergestellt. Schmelzpunkt: 159-160°C.

### Beispiel 4:

### 4-(5-Chlor-2-methoxy-benzamidomethyl)-6-(methylaminothiocarbonylaminosulfonyl)-7-methoxychroman

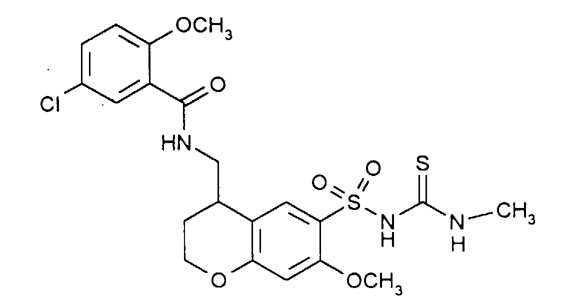

1.76 g (4 mmol) 4-(5-Chlor-2-methoxy-benzamidomethyl)-6-sulfamoyl-7-methoxychroman aus Beispiel 1 werden in 5 ml trockenem DMF gelöst und mit 1.65 g Kaliumkarbonat sowie mit 0.35 g (4.8 mmol) Methylisothiocyanat versetzt. Nach einstündigem Rühren bei 80°C wird abgekühlt und in Eiswasser eingetragen, mit Kohle geklärt und auf pH 1 angesäuert. Der Niederschlag wird abgesaugt, getrocknet und aus Ethanol/DMF umkristallisiert. Schmelzpunkt: 121°C.

### Beispiel 5:

### 4-(5-Chlor-2-methoxy-benzamidomethyl)-6-(ethylaminothiocarbonylaminosulfonyl)-7-methoxychroman

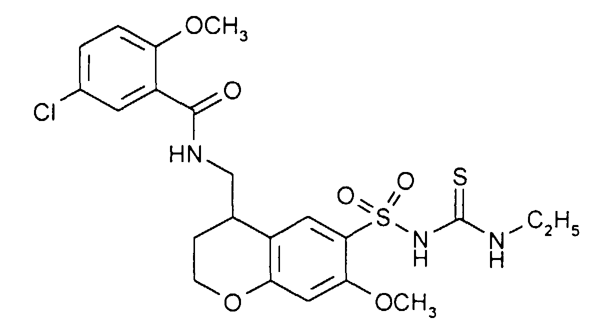

4-(5-Chlor-2-methoxy-benzamidomethyl)-6-(ethylaminothiocarbonylaminosulfonyl)-7-methoxychroman wird wie in Beispiel 4 beschrieben aus 4-(5-Chlor-2-methoxy-benzamidomethyl)-6-sulfamoyl-7-methoxychroman und Ethylisothiocyanat synthetisiert. Schmelzpunkt: 196-197°C.

### Beispiel 6:

### 4-(5-Chlor-2-methoxy-benzamidomethyl)-6-(n-propylaminothiocarbonylaminosulfonyl)-7-methoxychroman

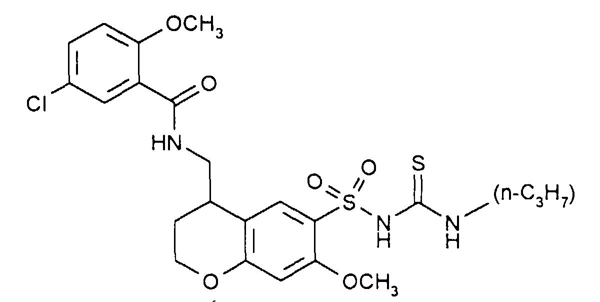

4-(5-Chlor-2-methoxy-benzamidomethyl)-6-(n-propylaminothiocarbonylaminosulfonyl)-7-methoxychroman wird wie in Beispiel 4 beschrieben aus 4-(5-Chlor-2-methoxy-benzamidomethyl)-6-sulfamoyl-7-methoxychroman und n-Propylisothiocyanat hergestellt. Schmelzpunkt: 183-184°C.

### Beispiel 7:

### 4-(5-Chlor-2-methoxy-benzamidomethyl)-6-(isopropylaminothiocarbonylaminosulfonyl)-7-methoxychroman

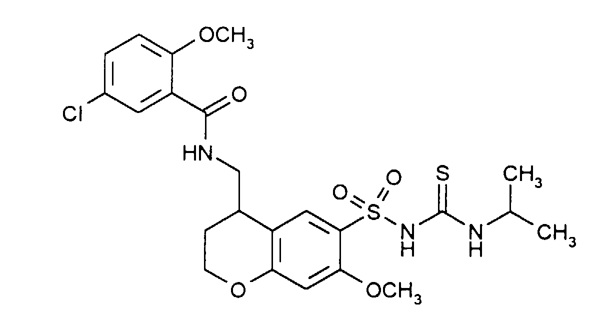

4-(5-Chlor-2-methoxy-benzamidomethyl)-6-(isopropylaminothiocarbonylaminosulfonyl)-7-methoxychroman wird analog zu Beispiel 4 aus 4-(5-Chlor-2-methoxy-benzamidomethyl)-6-sulfamoyl-7-methoxychroman und Isopropylisothiocyanat synthetisiert. Schmelzpunkt: 184-185°C.

### Beispiel 8:

### 4-(5-Chlor-2-methoxy-benzamidomethyl)-6-(n-butylaminothiocarbonylaminosulfonyl)-7-methoxychroman

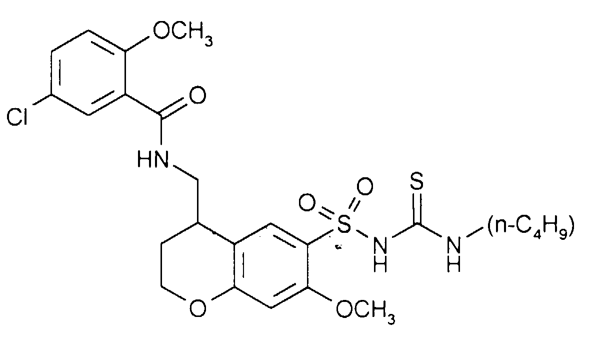

4-(5-Chlor-2-methoxy-benzamidomethyl)-6-(n-butylaminothiocarbonylaminosulfonyl)-7-methoxychroman wird analog Beispiel 4 aus 4-(5-Chlor-2-methoxybenzamidomethyl)-6-sulfamoyl-7-methoxychroman und n-Butylisothiocyanat hergestellt. Schmelzpunkt: 167°C.

### Beispiel 9:

### 4-(5-Chlor-2-methoxy-benzamidomethyl)-6-(methylaminocarbonylaminosulfonyl)-7-ethoxychroman

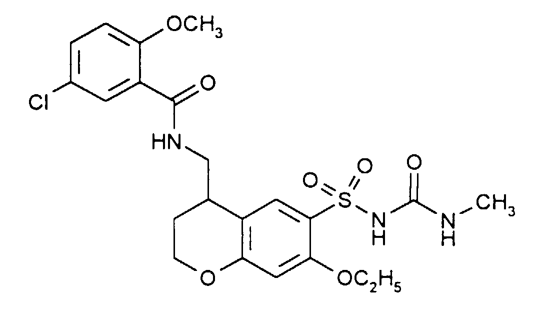

4-(5-Chlor-2-methoxy-benzamidomethyl)-6-(methylaminocarbonylaminosulfonyl)-7-ethoxychroman wird wie in Beispiel 1 beschrieben aus 4-(5-Chlor-2-methoxy-benzamidomethyl)-6-sulfamoyl-7-ethoxychroman und N-Methyltrichloracetamid dargestellt. Schmelzpunkt: 207-208°C.

### Herstellung der Ausgangsverbindung 4-(5-Chlor-2-methoxy-benzamidomethyl)-6-sulfamoyl-7-ethoxychroman

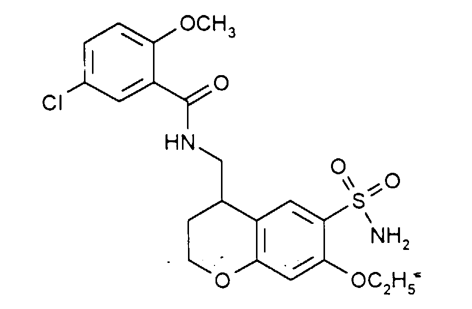

4-(5-Chlor-2-methoxy-benzamidomethyl)-6-sulfamoyl-7-ethoxychroman wird analog zur in Beispiel 1 beschriebenen Ausgangsverbindung, ausgehend von 4-Aminomethyl-7-ethoxychroman und 5-Chlor-2-methoxy-benzoesäurechlorid, hergestellt. Das so entstandene Zwischenprodukt wird anschließend mit Chlorsulfonsäure und danach mit Ammoniak umgesetzt. Man erhält 4-(5-Chlor-2-methoxybenzamidomethyl)-6-sulfamoyl-7-ethoxychroman.
Schmelzpunkt: 204-205°C.

### Beispiel 10:

### 4-(5-Chlor-2-methoxy-benzamidomethyl)-6-(methylaminothiocarbonylaminosulfonyl)-7-ethoxychroman

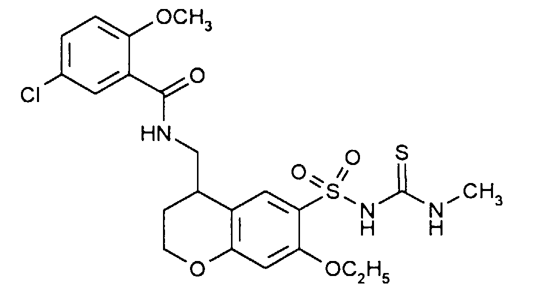

4-(5-Chlor-2-methoxy-benzamidomethyl)-6-(methylaminothiocarbonylaminosulfonyl)-7-ethoxychroman wird wie in Beispiel 4 beschrieben aus 4-(5-Chlor-2-methoxy-benzamidomethyl)-6-sulfamoyl-7-ethoxychroman und Methylisothiocyanat hergestellt. Schmelzpunkt: 202°C.

### Beispiel 11:

### 4-(5-Fluor-2-methoxy-benzamidomethyl)-6-(methylaminocarbonylaminosulfonyl)-7-methoxychroman

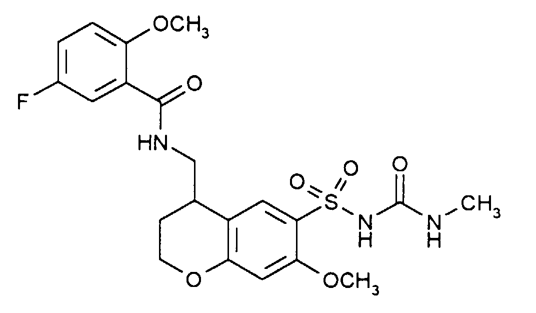

4-(5-Fluor-2-methoxy-benzamidomethyl)-6-(methylaminocarbonylaminosulfonyl)-7-methoxychroman wird analog Beispiel 1 aus 4-(5-Fluor-2-methoxybenzamidomethyl)-6-sulfamoyl-7-methoxychroman und N-Methyltrichloracetamid synthetisiert. Schmelzpunkt: 193-194°C.

### Herstellung der Ausgangsverbindung 4-(5-Fluor-2-methoxy-benzamidomethyl)-6-sulfamoyl-7-methoxychroman

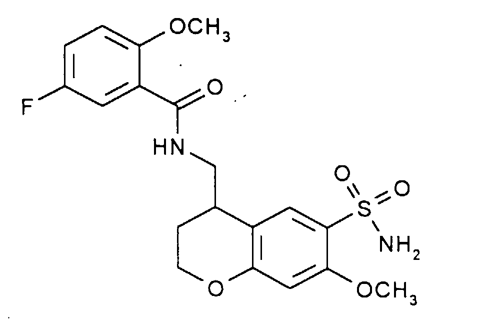

4-(5-Fluor-2-methoxy-benzamidomethyl)-6-sulfamoyl-7-methoxychroman wird analog zu Beispiel 1, ausgehend von 4-Aminomethyl-7-methoxychroman und 2-Methoxy-5-fluorbenzoesäure, hergestellt. Das Zwischenprodukt wird - wie in Beispiel 1 beschrieben - sulfochloriert und anschließend mit Ammoniak zur entsprechenden Sulfamoyl-Verbindung umgesetzt. Schmelzpunkt: 206°C.

### Beispiel 12:

### 4-(5-Fluor-2-methoxy-benzamidomethyl)-6-(methylaminothiocarbonylaminosulfonyl)-7-methoxychroman

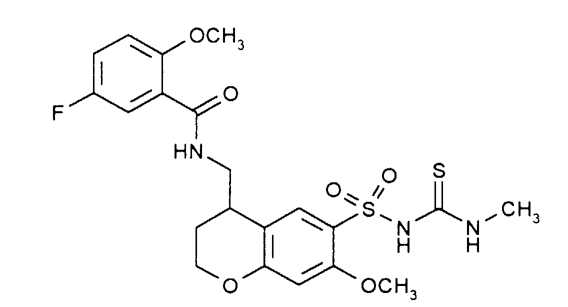

4-(5-Fluor-2-methoxy-benzamidomethyl)-6-(methylaminothiocarbonylaminosulfonyl)-7-methoxychroman wird analog zu Beispiel 4 durch Umsetzung von 4-(5-Fluor-2-methoxy-benzamidomethyl)-6-sulfamoyl-7-methoxychroman und Methylisothiocyanat erhalten. Schmelzpunkt: 194°C.

### Beispiel 13:

### 4-(5-Fluor-2-methoxy-benzamidomethyl)-6-(ethylaminothiocarbonylaminosulfonyl)-7-methoxychroman

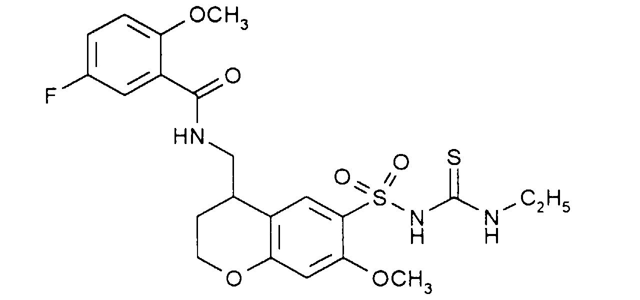

4-(5-Fluor-2-methoxy-benzamidomethyl)-6-(ethylaminothiocarbonylaminosulfonyl)-7-methoxychroman wird wie in Beispiel 4 beschrieben aus 4-(5-Fluor-2-methoxy-benzamidomethyl)-6-sulfamoyl-7-methoxychroman und Ethylisothiocyanat erhalten. Schmelzpunkt: 207°C.

### Beispiel 14:

### 4-((3-Ethyl-4-methyl-2-oxo-3-pyrrolin-1-carboxamido)methyl)-6-(methylaminothiocarbonylaminosulfonyl)-7-methoxychroman

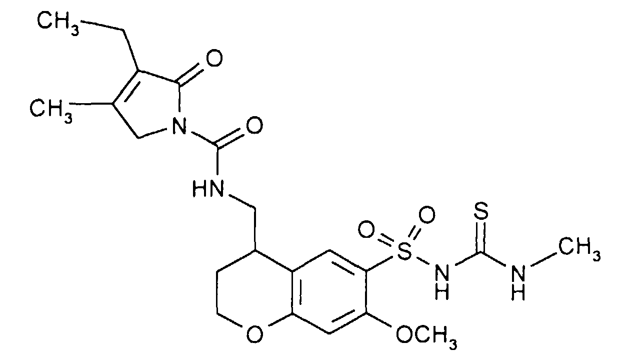

1.27 g (3 mmol) 4-((3-Ethyl-4-methyl-2-oxo-3-pyrrolin-1-carboxamido)methyl)-6-sulfamoyl-7-methoxychroman, 1.24 g (9 mmol) fein gepulvertes Kaliumkarbonat und 0.292 g (4 mmol) Methylisothiocyanat werden in 12 ml DMSO suspendiert bzw. gelöst. Das Reaktionsgemisch wird 1 Stunde bei 80°C gerührt. Der Ansatz wird auf Eiswasser gegossen und das Produkt durch Ansäuern mit Salzsäure ausgefällt. Nach Absaugen und Trocknen wird das Rohprodukt durch Chromatographie an Kieselgel (Elutionsmittel : Methylenchlorid/Eisessig 9:1) gereinigt.
Schmelzpunkt: 115°C.

### Herstellung der Ausgangsverbindung 4-((3-Ethyl-4-methyl-2-oxo-3-pyrrolin-1-carboxamido)methyl)-6-sulfamoyl-7-methoxychroman

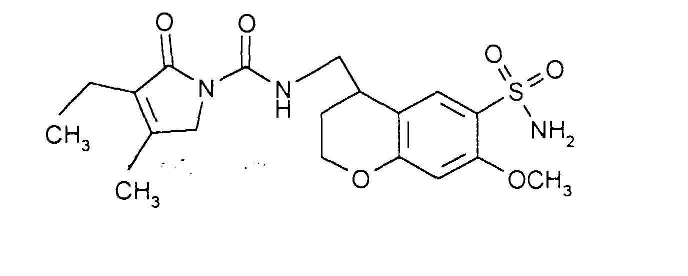

7.33 g (38 mmol) 4-Aminomethyl-7-methoxychroman werden in 50 ml Tetrahydrofuran gelöst. Dazu gibt man 6.16 g (38 mmol) N,N'-Carbonyldiimidazol. Es wird eine Stunde bei Raumtemperatur gerührt und dann im Vakuum eingedampft. In den Rückstand gibt man 4.76 g (38 mmol) 3-Ethyl-4-methyl-2-oxo-3-pyrrolin und erhitzt das Gemisch auf 160-170°C für 2 Stunden. Man chromatographiert an Kieselgel mit dem Elutionsmittel Essigester/Petrolether (3:1) und erhält 4-((3-Ethyl-4-methyl-2-oxo-3-pyrrolin-1-carboxamido)methyl)-7-methoxychroman vom Schmelzpunkt 115°C. Dieses Produkt wird in der üblichen Art und Weise in auf -15°C gekühlte Chlorsulfonsäure eingetragen. Man läßt auf Raumtemperatur kommen und rührt eine Stunde nach. Nach üblicher Aufarbeitung wird das Sulfochlorid wie in Beispiel 1 beschrieben in das Sulfonamid umgewandelt. 4-((3-Ethyl-4-methyl-2-oxo-3-pyrrolin-1-carboxamido)methyl)-6-sulfamoyl-7-methoxychroman hat einen Schmelzpunkt von 235-236°C.

### Beispiel 15:

### 4-((3-Ethyl-4-methyl-2-oxo-3-pyrrolin-1-carboxamido)methyl)-6-(ethylaminothiocarbonylaminosulfonyl)-7-methoxychroman

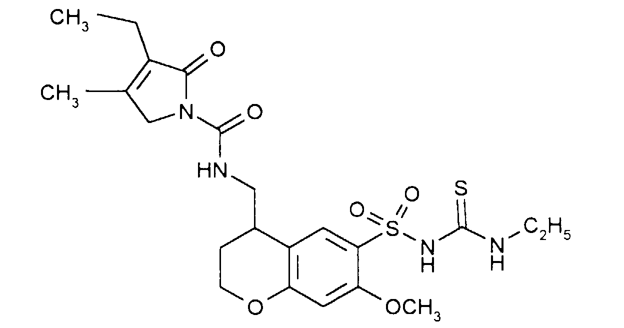

4-((3-Ethyl-4-methyl-2-oxo-3-pyrrolin-1-carboxamido)methyl)-6-(ethylaminothiocarbonylaminosulfonyl)-7-methoxychroman wird analog zu Beispiel 14 aus 4-((3-Ethyl-4-methyl-2-oxo-3-pyrrolin-1-carboxamido)methyl)-6-sulfamoyl-7-methoxychroman und Ethylisothiocyanat hergestellt. Schmelzpunkt: 147°C.

### Beispiel 16:

### 4-((3-Ethyl-4-methyl-2-oxo-3-pyrrolin-1-carboxamido)methyl)-6-(n-propylaminothiocarbonylaminosulfonyl)-7-methoxychroman

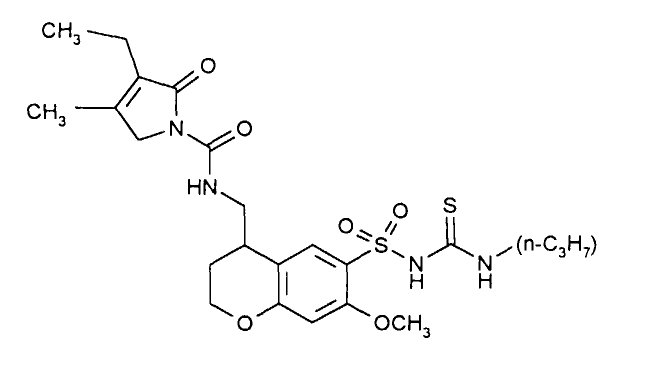

4-((3-Ethyl-4-methyl-2-oxo-3-pyrrolin-1-carboxamido)methyl)-6-(n-propylaminothiocarbonylaminosulfonyl)-7-methoxychroman wird wie in Beispiel 14 beschrieben aus 4-((3-Ethyl-4-methyl-2-oxo-3-pyrrolin-1-carboxamido)methyl)-6-sulfamoyl-7-methoxychroman und n-Propylisothiocyanat dargestellt. Schmelzpunkt: 96-98°C.

### Beispiel 17:

### 4-((3-Ethyl-4-methyl-2-oxo-3-pyrrolin-1-carboxamido)methyl)-6-(isopropylaminothiocarbonylaminosulfonyl)-7-methoxychroman

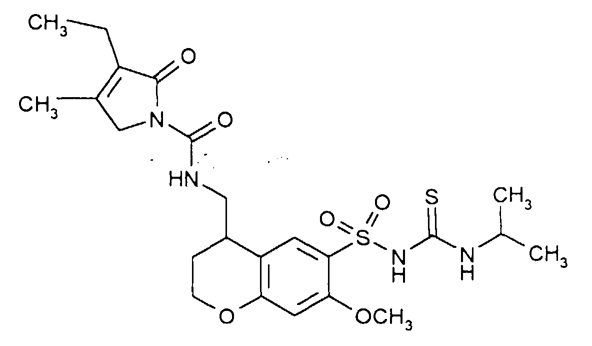

4-((3-Ethyl-4-methyl-2-oxo-3-pyrrolin-1-carboxamido)methyl)-6-(isopropylaminothiocarbonylaminosulfonyl)-7-methoxychroman wird in Anlehnung an Beispiel 14 ausgehend von 4-((3-Ethyl-4-methyl-2-oxo-3-pyrrolin-1-carboxamido)methyl)-6-sulfamoyl-7-methoxychroman und Isopropylisothiocyanat synthetisiert. Schmelzpunkt: 153°C.

### Beispiel 18:

### 4-((3-Ethyl-4-methyl-2-oxo-3-pyrrolin-1-carboxamido)methyl)-6-(methylaminocarbonylaminosulfonyl)-7-methoxychroman

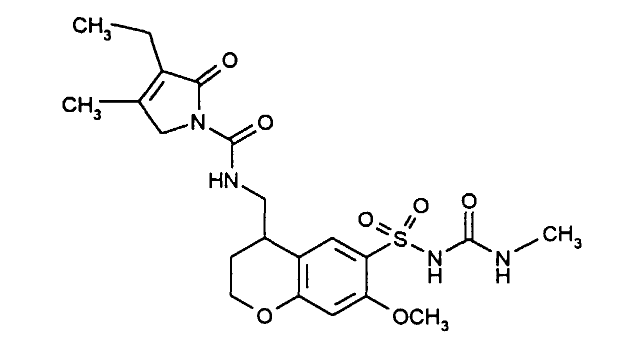

0.5 g 4-((3-Ethyl-4-methyl-2-oxo-3-pyrrolin-1-carboxamido)methyl)-6-(methylaminothiocarbonylaminosulfonyl)-7-methoxychroman (Beispiel 14) werden in 10 ml kalter 0.5N Natronlauge gelöst. In der Kälte (-4 bis 0°C) werden 0.5 ml 37%ige Wasserstoffperoxidlösung zugegeben und der Ansatz 1 Stunde bei 0°C gerührt. Das Produkt wird durch Zugabe von 2N HCI ausgefällt. Das Rohprodukt wird durch Chromatographie an Kieselgel (Elutionsmittel : Methylenchlorid/Eisessig 9:1) gereinigt. Schmelzpunkt: 211°C.

### Beispiel 19:

### 4-((3-Ethyl-4-methyl-2-oxo-3-pyrrolin-1-carboxamido)methyl)-6-(ethylaminocarbonylaminosulfonyl)-7-methoxychroman

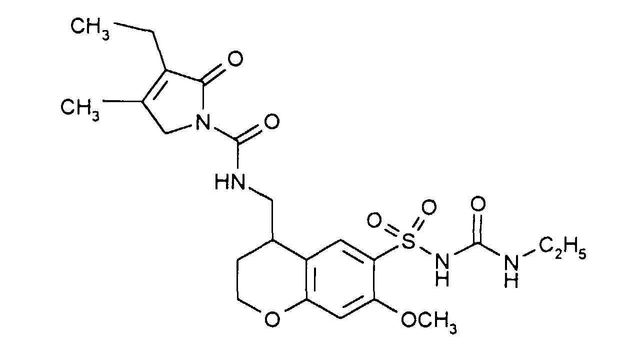

4-((3-Ethyl-4-methyl-2-oxo-3-pyrrolin-1-carboxamido)methyl)-6-(ethylaminothiocarbonylaminosulfonyl)-7-methoxychroman wird analog zu Beispiel 18 durch Oxidation von 4-((3-Ethyl-4-methyl-2-oxo-3-pyrrolidinyl-1-carboxamido)methyl)-6-(ethylaminothiocarbonylaminosulfonyl)-7-methoxychroman mit 37%iger Wasserstoffperoxidlösung gewonnen. Schmelzpunkt: 188-189°C.

### Beispiel 20:

### 4-((3-Ethyl-4-methyl-2-oxo-3-pyrrolin-1-carboxamido)methyl)-6-(methylaminothio-carbonylaminosulfonyl)-7-ethoxychroman

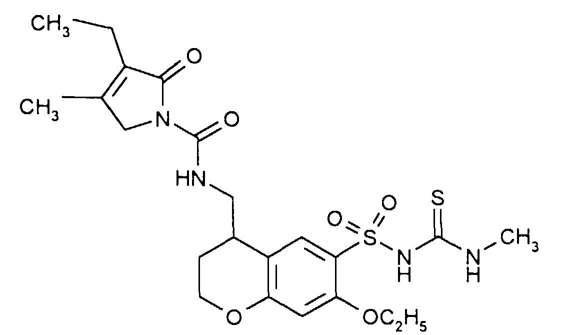

4-((3-Ethyl-4-methyl-2-oxo-3-pyrrolin-1-carboxamido)methyl)-6-(methylaminothiocarbonylaminosulfonyl)-7-ethoxychroman wird analog zu Beispiel 14 aus 4-((3-Ethyl-4-methyl-2-oxo-3-pyrrolin-1-carboxamido)methyl)-6-sulfamoyl-7-ethoxychroman und Methylisothiocyanat synthetisiert. Schmelzpunkt: 178°C.

### Herstellung der Ausgangsverbindung 4-((3-Ethyl-4-methyl-2-oxo-3-pyrrolin-1-carboxamido)methyl)-6-sulfamoyl-7-ethoxychroman

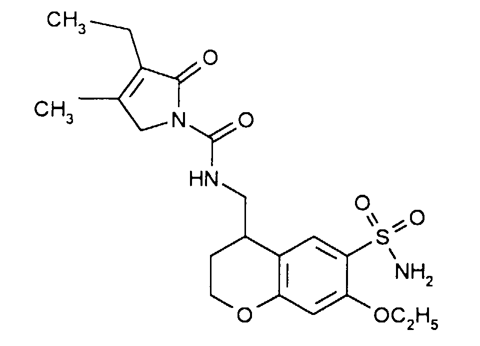

Zu einer auf -10°C abgekühlten Lösung von 10.6 g (0.025 Mol) 4-((3-Ethyl-4-methyl-2-oxo-3-pyrrolin-1-carboxamido)methyl)-6-sulfamoyl-7-methoxychroman in 75 ml Methylenchlorid werden 9.5 ml (0.1 Mol) Bortribromid zugetropft. Nach Stehen über Nacht bei 20°C wird das überschüssige Bortribromid nach Abkühlen auf -10°C durch vorsichtiges Zutropfen von Methanol zerstört. Anschließend wird in Eis/Wasser eingetragen und mehrfach mit Methylenchlorid extrahiert. Die vereinigten Methylenchloridextrakte werden getrocknet, eingedampft und aus Methanol umkristallisiert. Man erhält 4-((3-Ethyl-4-methyl-2-oxo-3-pyrrolin-1-carboxamido)methyl)-6-sulfamoyl-7-hydroxychroman vom Schmelzpunkt 175°C.

Zu einer Suspension von 9 g (0.022 Mol) 4-((3-Ethyl-4-methyl-2-oxo-3-pyrrolin-1-carboxamido)methyl)-6-sulfamoyl-7-hydroxychroman und 6.1 g (0.044 Mol) Kaliumkarbonat in 60 ml Aceton werden 2.16 ml (0.027 Mol) Ethyliodid zugegeben. Nach dreistündigem Rühren unter Rückfluß wird in Eis/Wasser eingetragen und mit konzentrierter Salzsäure vorsichtig angesäuert. Der Niederschlag wird abgesaugt, mehrfach mit kaltem Wasser gewaschen, getrocknet und aus Ethanol/DMF umkristallisiert. Man erhält 4-((3-Ethyl-4-methyl-2-oxo-3-pyrrolin-1-carboxamido)methyl)-6-sulfamoyl-7-ethoxychroman vom Schmelzpunkt 192°C.

### Beispiel 21:

### 4-((3-Ethyl-4-methyl-2-oxo-3-pyrrolin-1-carboxamido)methyl)-6-(ethylaminothiocarbonylaminosulfonyl)-7-ethoxychroman

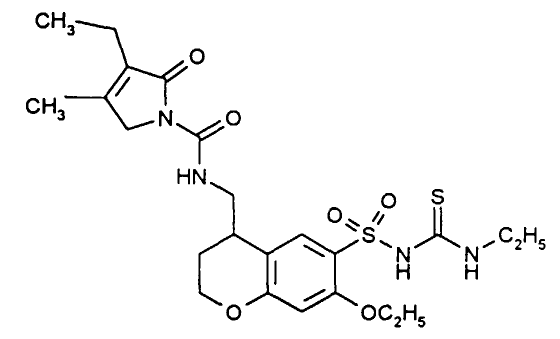

4-((3-Ethyl-4-methyl-2-oxo-3-pyrrolin-1-carboxamido)methyl)-6-(ethylaminothiocarbonylaminosulfonyl)-7-ethoxychroman wird analog zu Beispiel 14, ausgehend von 4-((3-Ethyl-4-methyl-2-oxo-3-pyrrolin-1-carboxamido)methyl)-6-sulfamoyl-7-ethoxychroman und Ethylisothiocyanat, synthetisiert. Schmelzpunkt: 178-180°C.

### Beispiel 22:

### 4-((3-Ethyl-4-methyl-2-oxo-3-pyrrolin-1-carboxamido)methyl)-6-(methylaminocarbonylaminosulfonyl)-7-ethoxychroman

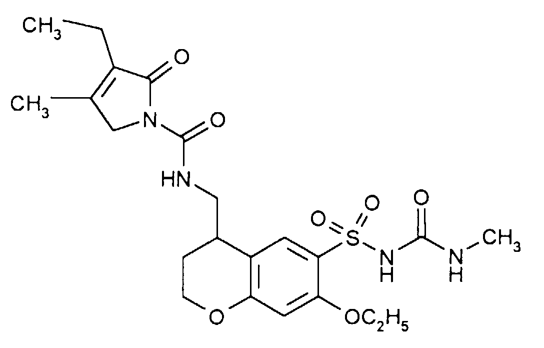

4-((3-Ethyl-4-methyl-2-oxo-3-pyrrolin-1-carboxamido)methyl)-6-(methylaminocarbonylaminosulfonyl)-7-ethoxychroman wird analog zu Beispiel 18 durch Oxidation von 4-((3-Ethyl-4-methyl-2-oxo-3-pyrrolin-1-carboxamido)methyl)-6-(methylaminothiocarbonylaminosulfonyl)-7-ethoxychroman mit Wasserstoffperoxidlösung hergestellt. Schmelzpunkt: 187-188°C.

### Beispiel 23:

### 4-((3-Ethyl-4-methyl-2-oxo-3-pyrrolin-1-carboxamido)methyl)-6-(ethylaminocarbonylaminosulfonyl)-7-ethoxychroman

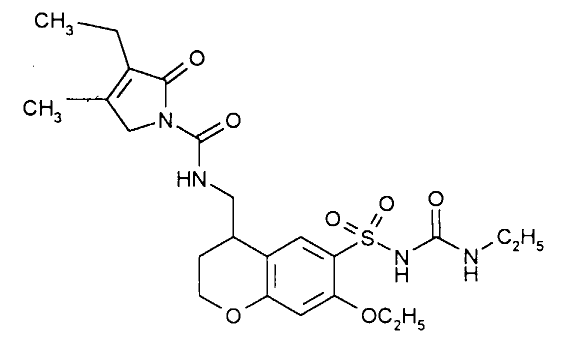

4-((3-Ethyl-4-methyl-2-oxo-3-pyrrolin-1-carboxamido)methyl)-6-(ethylaminocarbonylaminosulfonyl)-7-ethoxychroman wird analog zu Beispiel 18 durch Oxidation von 4-((3-Ethyl-4-methyl-2-oxo-3-pyrrolin-1-carboxamido)methyl)-6-(ethylaminothiocarbonylaminosulfonyl)-7-ethoxychroman mit Wasserstoffperoxidlösung gewonnen. Schmelzpunkt: 175°C.

### Beispiel 24:

### (+)-4-(5-Chlor-2-methoxy-benzamidomethyl)-6-(methylaminocarbonylaminosulfonyl)-7-methoxychroman

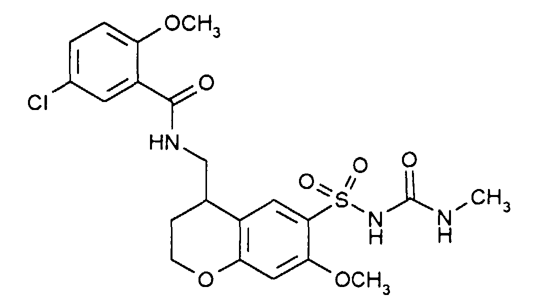

(+)-4-(5-Chlor-2-methoxy-benzamidomethyl)-6-(methylaminocarbonylaminosulfonyl)-7-methoxychroman wird analog zu Beispiel 1 aus optisch aktivem 4-(5-Chlor-2-methoxy-benzamidomethyl)-6-sulfamoyl-7-methoxychroman und N-Methyltrichloracetamid hergestellt. Schmelzpunkt: 242°C; [α]_{D}²⁰: +63.4° (c = 1, DMF); HPLC: ee 100 %.

Für die Synthese der Ausgangsverbindung, die wie in Beispiel 1 beschrieben hergestellt wird, wird das rechtsdrehende Mandelat des 4-Aminomethyl-7-methoxychromans (physikochemische Daten des Mandelats: Schmelzpunkt 144°C; [α]_{D}²⁰: +57.5° (c = 1, H₂O); HPLC: ee 93.8 %) eingesetzt.

### Beispiel 25:

### (+)-4-(5-Chlor-2-methoxy-benzamidomethyl)-6-(methylaminothiocarbonylaminosulfonyl)-7-methoxychroman

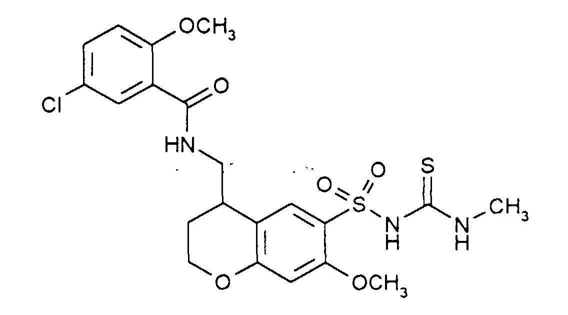

(+)-4-(5-Chlor-2-methoxy-benzamidomethyl)-6-(methylaminothiocarbonylaminosulfonyl)-7-methoxychroman wird wie in Beispiel 4 beschrieben aus optisch aktivem 4-(5-Chlor-2-methoxy-benzamidomethyl)-6-sulfamoyl-7-methoxychroman und Methylisothiocyanat hergestellt. Schmelzpunkt: 201 °C; [α]_{D}²⁰: +47.2° (c = 1, DMF); HPLC: ee 88.1 %.

### Beispiel 26:

### (-)-4-(5-Chlor-2-methoxy-benzamidomethyl)-6-(methylaminocarbonylaminosulfonyl)-7-methoxychroman

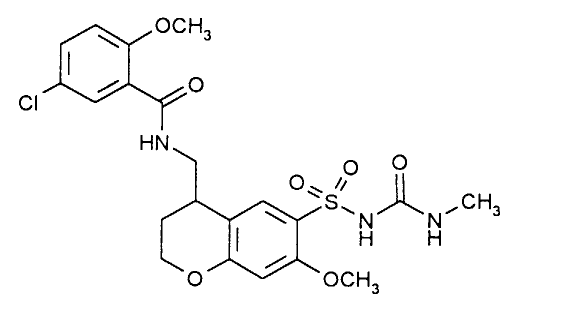

(-)-4-(5-Chlor-2-methoxy-benzamidomethyl)-6-(methylaminocarbonylaminosulfonyl)-7-methoxychroman wird analog zu Beispiel 1 aus optisch aktivem 4-(5-Chlor-2-methoxy-benzamidomethyl)-6-sulfamoyl-7-methoxychroman und N-Methyltrichloracetamid hergestellt. Schmelzpunkt: 239°C; [α]_{D}²⁰: -59.8° (c = 1, DMF); HPLC: ee 98.5 %.

Für die Synthese der Ausgangsverbindung, die wie in Beispiel 1 beschrieben hergestellt wird, wird das linksdrehende Mandelat des 4-Aminomethyl-7-methoxychromans (physikochemische Daten des Mandelats: Schmelzpunkt: 147-148°C; [α]_{D}²⁰: -59.5° (c = 1, H₂O); HPLC: ee 99.1 %) eingesetzt.

### Beispiel 27:

### (-)-4-(5-Chlor-2-methoxy-benzamidomethyl)-6-(methylaminothiocarbonylaminosulfonyl)-7-methoxychroman

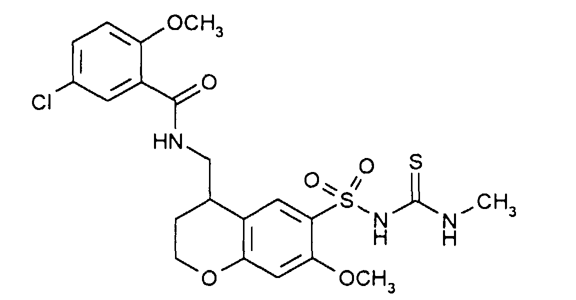

(-)-4-(5-Chlor-2-methoxy-benzamidomethyl)-6-(methylaminothiocarbonylaminosulfonyl)-7-methoxychroman wird wie in Beispiel 4 beschrieben aus optisch aktivem 4-(5-Chlor-2-methoxy-benzamidomethyl)-6-sulfamoyl-7-methoxychroman und Methylisothiocyanat hergestellt. Schmelzpunkt: 202°C; [α]_{D}²⁰: -64.5° (c = 1, DMF); HPLC: ee 97.9 %.

### Pharmakologische Daten

Mit dem folgenden Modell können die therapeutischen Eigenschaften der Verbindungen der Formel I nachgewiesen werden.

Aktionspotentialdauer am Papillarmuskel des Meerschweinchens:

### (a) Einleitung

ATP-Mangelzustände, wie sie während einer Ischämie in der Herzmuskelzelle beobachtet werden, führen zu einer Verkürzung der Aktionspotentialdauer. Sie gelten als eine der Ursachen für sogenannte Reentry-Arrhythmien, die den plötzlichen Herztod verursachen können. Die Öffnung von ATP-sensitiven K-Kanälen durch das Absinken von ATP gilt hierfür als ursächlich.

### (b) Methode

Zur Messung des Aktionspotentials wird eine Standard-Mikroelektroden-Technik eingesetzt. Hierfür werden Meerschweinchen beiderlei Geschlechts durch Schlag auf den Kopf getötet, die Herzen entnommen, die Papillarmuskeln herausgetrennt und in einem Organbad aufgehängt. Das Organbad wird mit Ringerlösung (0.9% NaCl, 0.048% KCl, 0.024% CaCl₂, 0.02% NaHCO₃, und 0.1% Glucose) durchspült und mit einer Mischung aus 95% Sauerstoff und 5% Kohlendioxid bei einer Temperatur von 36°C begast. Der Muskel wird über eine Elektrode mit Rechteck-Impulsen von 1 V und 1 ms Dauer und einer Frequenz von 2 Hz angeregt. Das Aktionspotential wird durch eine intrazellulär eingestochene Glas-Mikroelektrode, die mit 3 M KCl-Lösung gefüllt ist, abgeleitet und registriert. Die zu prüfenden Substanzen werden der Ringerlösung in einer Konzentration von 2.2·10⁻⁶ Mol pro Liter zugesetzt. Das Aktionspotential wird mit einem Amplifier von Hugo Sachs verstärkt auf einem Oszilloskop dargestellt. Die Dauer des Aktionspotentials wird bei einem Repolarisierungsgrad von 95% (APD₉₅) bestimmt. Aktionspotentialverkürzungen werden durch Zugabe einer 1 µM starken Lösung des Kaliumkanalöffners Hoe 234 (Rilmakalim) (W. Linz, E. Klaus, U. Albus, R.H.A. Becker, D. Mania, H.C. Englert, B.A. Schölkens, Arzneimittelforschung/Drug Research, Band 42 (II), 1992, S. 1180 - 1185) ausgelöst. Testsubstanzen wurden als Stammlösungen in Propandiol der Badlösung zugesetzt. Die angegebenen Werte beziehen sich auf Messungen 30 min nach der Zugabe. Als Kontrolle gilt die APD₉₅ in Gegenwart von HOE 234 und in Abwesenheit der Testsubstanz.

### (c) Resultate

Folgende Werte wurden gemessen:

| Messung | APD₉₅-HOE 234^{a)} [ms] |
|---|---|
| Kontrolle | < 40 |
| Beispiel 1 | 109 ± 1 (164 ± 18) n = 3 |
| Beispiel 2 | 96 ± 35 (141 ± 2) n = 3 |
| Beispiel 3 | 138 ± 8 (172 ± 11) n = 3 |
| Beispiel 4 | 144 ± 9 (181 ± 2) n = 3 |
| Beispiel 24 | 70 ± 7 (169 ± 10) n = 3 |
| Beispiel 25 | 123 ± 19 (158 ± 4) n = 3 |
| Beispiel 26 | 140 ± 7 (171 ± 14) n = 3 |
| Beispiel 27 | 169 ± 16 (164 ± 23) n = 3 |

| | |
|---|---|
| ^{a)} den Meßwerten (Mittelwert aus n Versuchen) sind die korrespondierenden Leerwerte in Klammern nachgestellt. Die Leerwerte sind die APD₉₅-Werte zu Versuchsbeginn ohne HOE 234 und Testsubstanz in der Ringerlösung. | |

## Patentansprüche

1. Chromanylsulfonyl(thio)harnstoffe der Formel I, 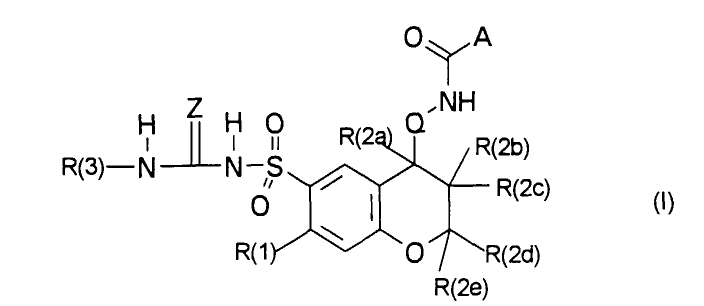 in welcher bedeuten:
R(1) Wasserstoff, Alkyl mit 1 bis 4 C-Atomen, Alkoxy mit 1 bis 4 C-Atomen, Fluor, Chlor, Brom, lod, CF₃, NH₂, NH-Alkyl mit 1 bis 4 C-Atomen, N(Alkyl)₂ mit 1 bis 4 C-Atomen in den gleichen oder verschiedenen Alkylresten, oder S-Alkyl mit 1 bis 4 C-Atomen;
R(2a) Wasserstoff oder Alkyl mit 1 oder 2 C-Atomen;
R(2b) und R(2d), die gleich oder verschieden sind, Wasserstoff, Alkyl mit 1 oder 2 C-Atomen, unsubstituiertes Phenyl, substituiertes Phenyl, unsubstituiertes Benzyl oder im Phenylrest substituiertes Benzyl, wobei als Substituenten in Phenylresten bis zu drei gleiche oder verschiedene Substituenten, ausgewählt aus der Gruppe bestehend aus Halogen, Alkyl mit 1 oder 2 C-Atomen, Alkoxy mit 1 oder 2 C-Atomen, vorhanden sind;
R(2c) und R(2e), die gleich oder verschieden sind, Wasserstoff oder Alkyl mit 1 oder 2 C-Atomen;
R(3) Wasserstoff, Alkyl mit 1, 2, 3 oder 4 C-Atomen, Cycloalkyl mit 3, 4, 5 oder 6 Ring-C-Atomen, CH₂-Cycloalkyl mit 3, 4, 5 oder 6 Ring-C-Atomen, oder CF₃;
Q (CH₂)ₙ;
n 1 oder 2;
Z Schwefel oder Sauerstoff;
A Phenyl, das unsubstituiert ist oder durch bis zu drei gleiche oder verschiedene Substituenten, ausgewählt aus der Gruppe bestehend aus Halogen, Alkyl mit 1 oder 2 C-Atomen, Alkoxy mit 1 oder 2 C-Atomen, substituiert ist;
oder
A den Rest eines gesättigten oder ungesättigten Lactams der Formel 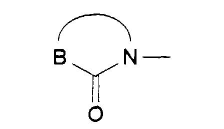
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen;
oder
A den Rest eines bicyclischen Systems der Formeln 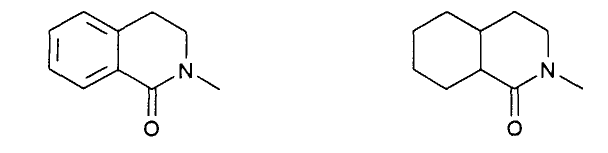 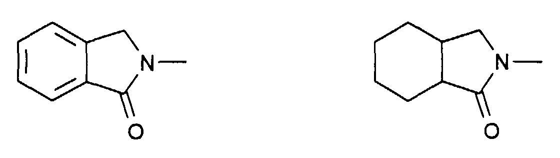
und deren physiologisch verträgliche Salze.

2. Verbindungen der Formel I nach Anspruch 1, in der bedeuten:
R(1) Wasserstoff, Alkyl mit 1 oder 2 C-Atomen, Alkoxy mit 1 bis 4 C-Atomen, Fluor, Chlor, Brom, lod oder CF₃;
R(2a), R(2b) und R(2d), die gleich oder verschieden sind, Wasserstoff oder Alkyl mit 1 oder 2 C-Atomen;
R(2c) und R(2e) Wasserstoff;
R(3) Wasserstoff oder Alkyl mit 1, 2, 3 oder 4 C-Atomen;
Q (CH₂)ₙ;
n 1 oder 2;
Z Schwefel oder Sauerstoff;
A Phenyl, das unsubstituiert ist oder durch bis zu drei gleiche oder verschiedene Substituenten, ausgewählt aus der Gruppe bestehend aus Halogen, Alkyl mit 1 oder 2 C-Atomen, Alkoxy mit 1 oder 2 C-Atomen, substituiert ist;
oder
A den Rest eines gesättigten oder ungesättigten Lactams der Formel 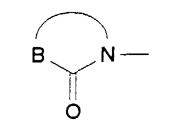
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen;
oder
A den Rest eines bicyclischen Systems der Formeln 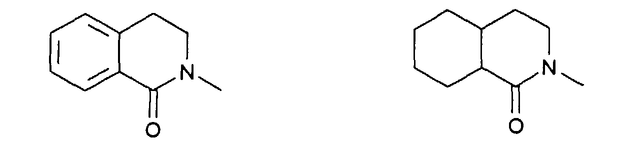 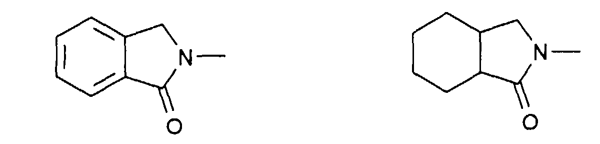
und deren physiologisch verträgliche Salze.

3. Verbindungen der Formel I nach Anspruch 1 oder 2, in der bedeuten:
R(1) Wasserstoff, Alkyl mit 1 oder 2 C-Atomen, Alkoxy mit 1 oder 2 C-Atomen, Fluor, Chlor, Brom, lod oder CF₃;
R(2a), R(2b), R(2c), R(2d), R(2e) Wasserstoff;
R(3) Wasserstoff oder Alkyl mit 1, 2, 3 oder 4 C-Atomen;
Q (CH₂)ₙ;
n 1 oder 2;
Z Schwefel oder Sauerstoff;
A Phenyl, das unsubstituiert ist oder durch bis zu drei gleiche oder verschiedene Substituenten, ausgewählt aus der Gruppe bestehend aus Halogen, Alkyl mit 1 oder 2 C-Atomen, Alkoxy mit 1 oder 2 C-Atomen, substituiert ist;
oder
A den Rest eines gesättigten oder ungesättigten Lactams der Formel 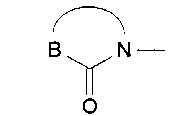
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen;
oder
A den Rest eines bicyclischen Systems der Formeln 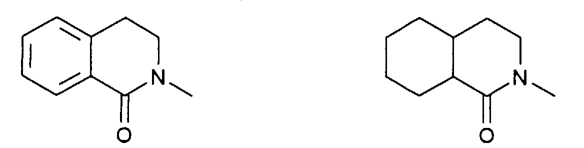 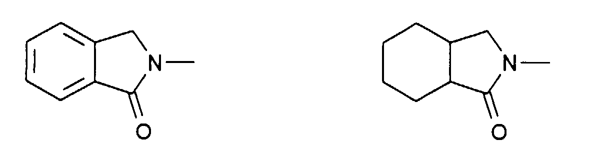
und deren physiologisch verträgliche Salze.

4. Verbindungen der Formel I nach mindestens einem der Ansprüche 1 bis 3, in der bedeuten:
R(1) Wasserstoff, Alkyl mit 1 oder 2 C-Atomen oder Alkoxy mit 1 oder 2 C-Atomen;
R(2a), R(2b), R(2c), R(2d), R(2e) Wasserstoff;
R(3) Wasserstoff oder Alkyl mit 1, 2, 3 oder 4 C-Atomen;
Q (CH₂)ₙ;
n 1 oder 2;
Z Schwefel;
A Phenyl, das unsubstituiert ist oder durch bis zu drei gleiche oder verschiedene Substituenten, ausgewählt aus der Gruppe bestehend aus Halogen, Alkyl mit 1 oder 2 C-Atomen, Alkoxy mit 1 oder 2 C-Atomen, substituiert ist;
oder
A den Rest eines gesättigten oder ungesättigten Lactams der Formel 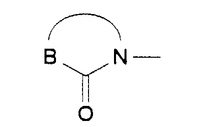
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen;
oder
A den Rest eines bicyclischen Systems der Formeln 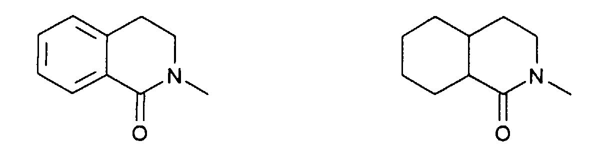 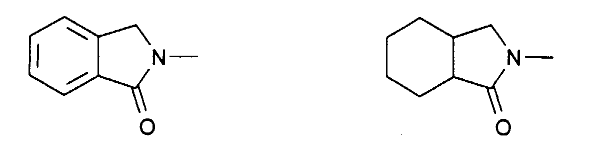
und deren physiologisch verträgliche Salze.

5. Verbindungen der Formel I nach mindestens einem der Ansprüche 1 bis 4, in der bedeuten:
R(1) Wasserstoff, Alkyl mit 1 oder 2 C-Atomen oder Alkoxy mit 1 oder 2 C-Atomen;
R(2a), R(2b), R(2c), R(2d), R(2e) Wasserstoff;
R(3) Wasserstoff, Methyl oder Ethyl;
Q (CH₂)ₙ;
n 1 oder 2;
Z Schwefel;
A Phenyl, das unsubstituiert oder durch bis zu drei gleiche oder verschiedene Substituenten, ausgewählt aus der Gruppe bestehend aus Halogen, Alkyl mit 1 oder 2 C-Atomen, Alkoxy mit 1 oder 2 C-Atomen, substituiert ist;
oder
A den Rest eines gesättigten oder ungesättigten Lactams der Formel 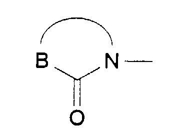
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen;
und deren physiologisch verträgliche Salze.

6. Verbindungen der Formel I nach mindestens einem der Ansprüche 1 bis 5, in der bedeuten:
R(1) Wasserstoff, Alkyl mit 1 oder 2 C-Atomen oder Alkoxy mit 1 oder 2 C-Atomen;
R(2a), R(2b), R(2c), R(2d), R(2e) Wasserstoff;
R(3) Wasserstoff, Methyl oder Ethyl;
Q CH₂;
Z Schwefel;
A Phenyl, das unsubstituiert oder durch bis zu drei gleiche oder verschiedene Substituenten ausgewählt aus der Gruppe bestehend aus Halogen, Alkyl mit 1 oder 2 C-Atomen, Alkoxy mit 1 oder 2 C-Atomen substituiert ist;
und deren physiologisch verträgliche Salze..

7. Verbindungen der Formel I nach mindestens einem der Ansprüche 1 bis 3, in der bedeuten:
R(1) Wasserstoff, Alkyl mit 1 oder 2 C-Atomen oder Alkoxy mit 1 oder 2 C-Atomen;
R(2a), R(2b), R(2c), R(2d), R(2e) Wasserstoff;
R(3) Wasserstoff oder Alkyl mit 1, 2, 3 oder 4 C-Atomen;
Q (CH₂)ₙ;
n 1 oder 2;
Z Sauerstoff;
A Phenyl, das unsubstituiert ist oder durch bis zu drei gleiche oder verschiedene Substituenten, ausgewählt aus der Gruppe bestehend aus Halogen, Alkyl mit 1 oder 2 C-Atomen, Alkoxy mit 1 oder 2 C-Atomen, substituiert ist;
oder
A den Rest eines gesättigten oder ungesättigten Lactams der Formel 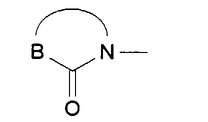
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen;
oder
A den Rest eines bicyclischen Systems der Formeln 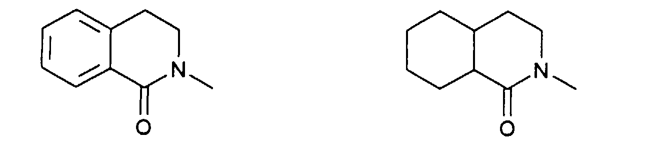 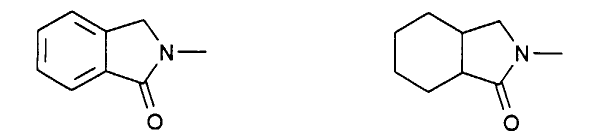
und deren physiologisch verträgliche Salze.

8. Verbindungen der Formel I nach mindestens einem der Ansprüche 1, 2, 3 und 7, in der bedeuten:
R(1) Wasserstoff, Alkyl mit 1 oder 2 C-Atomen oder Alkoxy mit 1 oder 2 C-Atomen;
R(2a), R(2b), R(2c), R(2d), R(2e) Wasserstoff;
R(3) Wasserstoff, Methyl oder Ethyl;
Q (CH₂)ₙ;
n 1 oder 2;
Z Sauerstoff;
A Phenyl, das unsubstituiert ist oder durch bis zu drei gleiche oder verschiedene Substituenten, ausgewählt aus der Gruppe bestehend aus Halogen, Alkyl mit 1 oder 2 C-Atomen, Alkoxy mit 1 oder 2 C-Atomen substituiert ist;
oder
A den Rest eines gesättigten oder ungesättigten Lactams der Formel 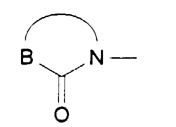
B Alkenylen oder Alkylen mit 3, 4, 5 oder 6 C-Atomen, das unsubstituiert ist oder substituiert ist durch bis zu drei gleiche oder verschiedene Alkylgruppen mit 1, 2, 3 oder 4 C-Atomen;
und deren physiologisch verträgliche Salze.

9. Verbindungen der Formel I nach mindestens einem der Ansprüche 1, 2, 3, 7 und 8, in der bedeuten:
R(1) Wasserstoff, Alkyl mit 1 oder 2 C-Atomen oder Alkoxy mit 1 oder 2 C-Atomen;
R(2a), R(2b), R(2c), R(2d), R(2e) Wasserstoff;
R(3) Wasserstoff, Methyl oder Ethyl;
Q CH₂;
Z Sauerstoff;
A Phenyl, das unsubstituiert ist oder durch bis zu drei gleiche oder verschiedene Substituenten, ausgewählt aus der Gruppe bestehend aus Halogen, Alkyl mit 1 oder 2 C-Atomen, Alkoxy mit 1 oder 2 C-Atomen, substituiert ist;
und deren physiologisch verträgliche Salze.

10. Verfahren zur Herstellung von Verbindungen der Formel I nach einem oder mehreren der Ansprüche 1 bis 9, dadurch gekennzeichnet, daß man Sulfamoylchromane der Formel II 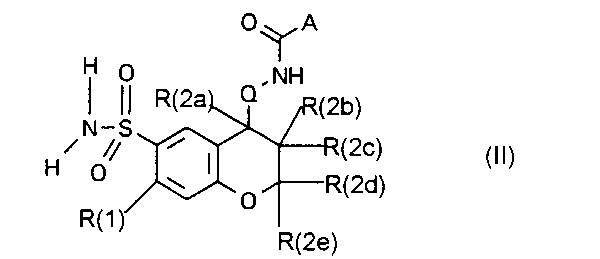 oder deren Salze der Formel III, 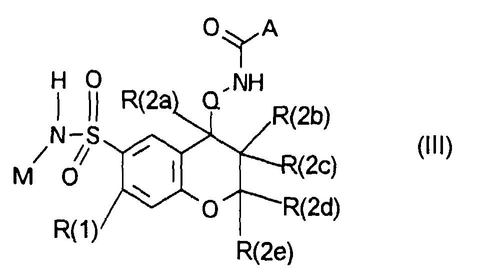 in denen die Reste die in den Ansprüchen 1 bis 9 angegebenen Bedeutungen haben und das Kation M beispielsweise für ein Alkali-, Erdalkali-, Ammonium- oder Tetraalkylammoniumion steht, mit einem R(3)-substituierten Isocyanat oder Isothiocyanat, einem R(3)-substituierten Kohlensäurederivat oder einem am Stickstoff R(3)-substituierten Trichloracetamid umsetzt;
oder, zur Herstellung von Verbindungen der Formel I, in denen R(3) für Wasserstoff steht, Verbindungen der Formeln II oder III mit einem Trialkylsilyliso(thio)cyanat oder Siliciumtetraiso(thio)cyanat umsetzt und die primär entstehenden siliciumsubstituierten Chromanylsulfonyl(thio)harnstoffe spaltet;
oder, zur Herstellung von Verbindungen der Formel I, in der Z für Sauerstoff steht, Verbindungen der Formel I, in der Z für Schwefel steht, entschwefelt;
oder, zur Herstellung von Verbindungen der Formel I, in der Z für Sauerstoff steht, Verbindungen der Formel VII, 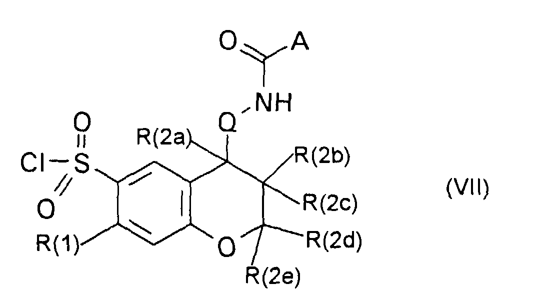 in der die Reste die in den Ansprüchen 1 bis 9 angegebenen Bedeutungen haben, mit einem R(3)-substituierten Harnstoff oder Bis(trialkyl)silylharnstoff umsetzt;
oder Verbindungen der Formel VIII oder der Formel IX, 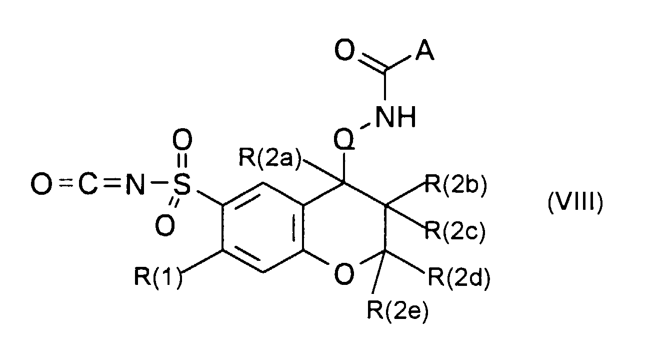 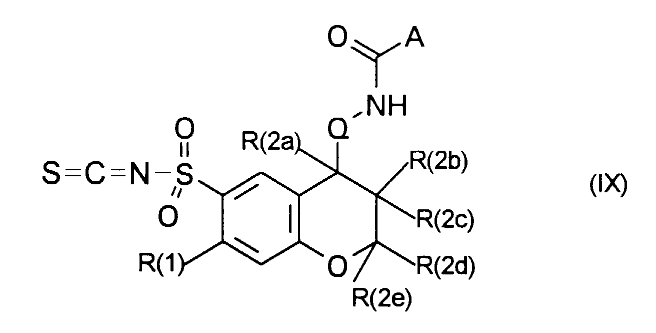 in denen die Reste die in den Ansprüchen 1 bis 9 angegebenen Bedeutungen haben, mit einem Amin der Formel R(3)-NH₂ umsetzt.

11. Verwendung einer Verbindung der Formel I nach einem oder mehreren der Ansprüche 1 bis 9 und/oder eines physiologisch verträglichen Salzes davon zur Herstellung eines Medikaments zur Behandlung von Herzrhythmusstörungen.

12. Verwendung einer Verbindung der Formel I nach einem oder mehreren der Ansprüche 1 bis 9 und/oder eines physiologisch verträglichen Salzes davon zur Herstellung eines Medikaments zur Verhinderung des plötzlichen Herztods.

13. Verwendung einer Verbindung der Formel I nach einem oder mehreren der Ansprüche 1 bis 9 und/oder eines physiologisch verträglichen Salzes davon zur Herstellung eines Medikaments zur Behandlung von ischämischen Zuständen des Herzens.

14. Verwendung einer Verbindung der Formel I nach einem oder mehreren der Ansprüche 1 bis 9 und/oder eines physiologisch verträglichen Salzes davon zur Herstellung eines wissenschaftliches Tools zur Inhibition ATP-sensitiver Kaliumkanäle.

15. Verwendung einer Verbindung der Formel I nach einem oder mehreren der Ansprüche 1 bis 9 und/oder eines physiologisch verträglichen Salzes davon zur Herstellung eines Medikaments zur Behandlung der geschwächten Herzkraft.

16. Verwendung einer Verbindung der Formel I nach einem oder mehreren der Ansprüche 1 bis 9 und/oder eines physiologisch verträglichen Salzes davon zur Herstellung eines Medikaments zur Verbesserung der Herzfunktion nach Herztransplantation.

17. Verbindung der Formel I nach einem oder mehreren der Ansprüche 1 bis 9 und/oder ein physiologisch verträgliches Salz davon zur Verwendung als Arzneimittel.

18. Arzneimittel, gekennzeichnet durch eine wirksame Menge an mindestens einer Verbindung der Formel I nach einem oder mehreren der Ansprüche 1 bis 9 und/oder eines physiologisch verträglichen Salzes davon.

## Claims

1. A chromanylsulfonyl(thio)urea of the formula I 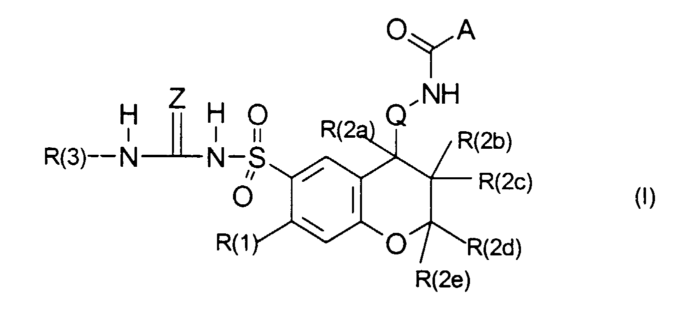 in which:
R(1) is hydrogen, alkyl having 1 to 4 carbon atoms, alkoxy having 1 to 4 carbon atoms, fluorine, chlorine, bromine, iodine, CF₃, NH₂, NH-alkyl having 1 to 4 carbon atoms, N(alkyl)₂ having 1 to 4 carbon atoms in the identical or different alkyl radicals, or S-alkyl having 1 to 4 carbon atoms;
R(2a) is hydrogen or alkyl having 1 or 2 carbon atoms;
R(2b) and R(2d), which are identical or different, are hydrogen, alkyl having 1 or 2 carbon atoms, unsubstituted phenyl, substituted phenyl, unsubstituted benzyl or benzyt substituted in the phenyl radical, up to three identical or different substituents selected from the group consisting of halogen, alkyl having 1 or 2 carbon atoms and alkoxy having 1 or 2 carbon atoms being present as substituents in phenyl radicals;
R(2c) and R(2e), which are identical or different, are hydrogen or alkyl having 1 or 2 carbon atoms;
R(3) is hydrogen, alkyl having 1, 2, 3 or 4 carbon atoms, cycloalkyl having 3, 4, 5 or 6 ring carbon atoms, CH₂-cycloalkyl having 3, 4, 5 or 6 ring carbon atoms, or CF₃;
Q is (CH₂)ₙ;
n is 1 or 2;
Z is sulfur or oxygen;
A is phenyl, which is unsubstituted or is substituted by up to three identical or different substituents selected from the group consisting of halogen, alkyl having 1 or 2 carbon atoms and alkoxy having 1 or 2 carbon atoms;
or
A is the radical of a saturated or unsaturated lactam of the formula 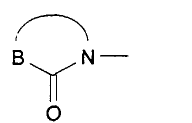
B is alkenylene or alkylene having 3, 4, 5 or 6 carbon atoms, which is unsubstituted or is substituted by up to three identical or different alkyl groups having 1, 2, 3 or 4 carbon atoms;
or
A is the radical of a bicyclic system of the formula 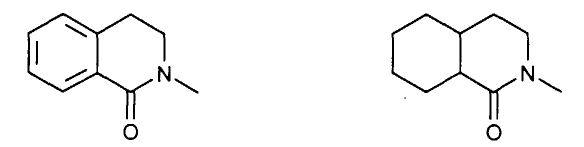 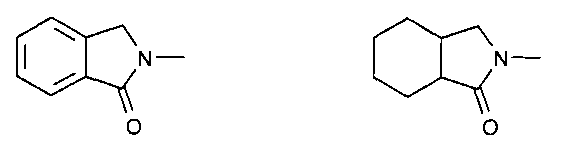
or its physiologically tolerable salts.

2. A compound of the formula I as claimed in claim 1, in which:
R(1) is hydrogen, alkyl having 1 or 2 carbon atoms, alkoxy having 1 to 4 carbon atoms, fluorine, chlorine, bromine, iodine or CF₃;
R(2a), R(2b) and R(2d), which are identical or different, are hydrogen or alkyl having 1 or 2 carbon atoms;
R(2c) and R(2e) are hydrogen;
R(3) is hydrogen or alkyl having 1, 2, 3 or 4 carbon atoms;
Q is (CH₂)ₙ;
n is 1 or 2;
Z is sulfur or oxygen;
A is phenyl, which is unsubstituted or is substituted by up to three identical or different substituents selected from the group consisting of halogen, alkyl having 1 or 2 carbon atoms and alkoxy having 1 or 2 carbon atoms;
or
A is the radical of a saturated or unsaturated lactam of the formula 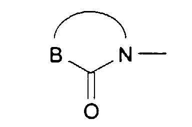
B is alkenylene or alkylene having 3, 4, 5 or 6 carbon atoms, which is unsubstituted or is substituted by up to three identical or different alkyl groups having 1, 2, 3 or 4 carbon atoms;
or
A is the radical of a bicyclic system of the formula 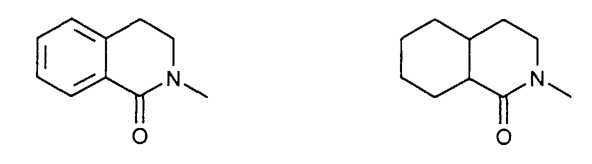 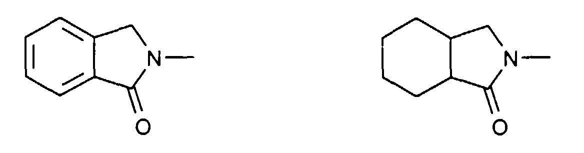
or its physiologically tolerable salts.

3. A compound of the formula I as claimed in claim 1 or 2, in which:
R(1) is hydrogen, alkyl having 1 or 2 carbon atoms, alkoxy having 1 or 2 carbon atoms, fluorine, chlorine, bromine, iodine or CF₃;
R(2a), R(2b), R(2c), R(2d) and R(2e) are hydrogen;
R(3) is hydrogen or alkyl having 1, 2, 3 or 4 carbon atoms;
Q is (CH₂)ₙ;
n is 1 or 2;
Z is sulfur or oxygen;
A is phenyl, which is unsubstituted or is substituted by up to three identical or different substituents selected from the group consisting of halogen, alkyl having 1 or 2 carbon atoms and alkoxy having 1 or 2 carbon atoms;
or
A is the radical of a saturated or unsaturated lactam of the formula 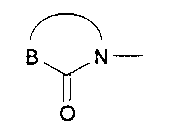
B is alkenylene or alkylene having 3, 4, 5 or 6 carbon atoms, which is unsubstituted or is substituted by up to three identical or different alkyl groups having 1, 2, 3 or 4 carbon atoms;
or
A is the radical of a bicyclic system of the formula 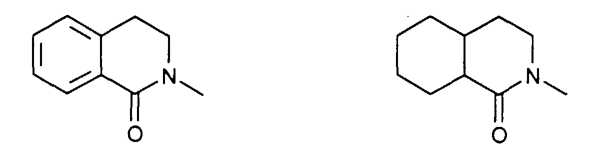 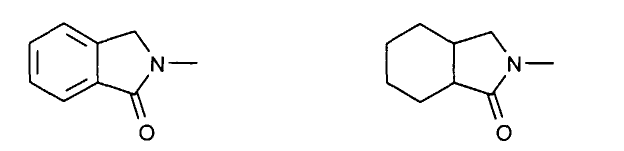
or its physiologically tolerable salts.

4. A compound of the formula I according to at least one of claims 1 to 3, in which:
R(1) is hydrogen, alkyl having 1 or 2 carbon atoms or alkoxy having 1 or 2 carbon atoms;
R(2a), R(2b), R(2c), R(2d) and R(2e) are hydrogen;
R(3) is hydrogen or alkyl having 1, 2, 3 or 4 carbon atoms;
Q is (CH₂)ₙ;
n is 1 or 2;
Z is sulfur;
A is phenyl, which is unsubstituted or is substituted by up to three identical or different substituents selected from the group consisting of halogen, alkyl having 1 or 2 carbon atoms and alkoxy having 1 or 2 carbon atoms;
or
A is the radical of a saturated or unsaturated lactam of the formula 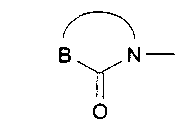
B is alkenylene or alkylene having 3, 4, 5 or 6 carbon atoms, which is unsubstituted or is substituted by up to three identical or different alkyl groups having 1, 2, 3 or 4 carbon atoms;
or
A is the radical of a bicyclic system of the formula 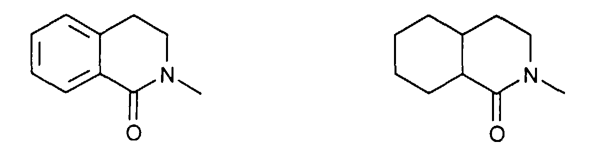 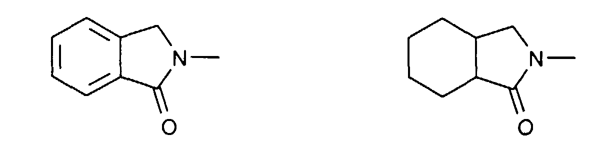
or its physiologically tolerable salts.

5. A compound of the formula I as claimed in at least one of claims 1 to 4, in which:
R(1) is hydrogen, alkyl having 1 or 2 carbon atoms or alkoxy having 1 or 2 carbon atoms;
R(2a), R(2b), R(2c), R(2d) and R(2e) are hydrogen;
R(3) is hydrogen, methyl or ethyl;
Q is (CH₂)ₙ;
n is 1 or 2;
Z is sulfur;
A is phenyl, which is unsubstituted or is substituted by up to three identical or different substituents selected from the group consisting of halogen, alkyl having 1 or 2 carbon atoms and alkoxy having 1 or 2 carbon atoms;
or
A is the radical of a saturated or unsaturated lactam of the formula 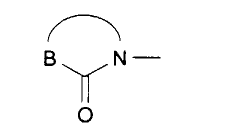
B is alkenylene or alkylene having 3, 4, 5 or 6 carbon atoms, which is unsubstituted or is substituted by up to three identical or different alkyl groups having 1, 2, 3 or 4 carbon atoms
or its physiologically tolerable salts.

6. A compound of the formula I as claimed in at least one of claims 1 to 5, in which:
R(1) is hydrogen, alkyl having 1 or 2 carbon atoms or alkoxy having 1 or 2 carbon atoms;
R(2a), R(2b), R(2c), R(2d) and R(2e) are hydrogen;
R(3) is hydrogen, methyl or ethyl;
Q is CH₂;
Z is sulfur;
A is phenyl, which is unsubstituted or is substituted by up to three identical or different substituents selected from the group consisting of halogen, alkyl having 1 or 2 carbon atoms and alkoxy having 1 or 2 carbon atoms
or its physiologically tolerable salts.

7. A compound of the formula I as claimed in at least one of claims 1 to 3, in which:
R(1) is hydrogen, alkyl having 1 or 2 carbon atoms or alkoxy having 1 or 2 carbon atoms;
R(2a), R(2b), R(2c), R(2d) and R(2e) are hydrogen;
R(3) is hydrogen or alkyl having 1, 2, 3 or 4 carbon atoms;
Q is (CH₂)ₙ;
n is 1 or 2;
Z is oxygen;
A is phenyl, which is unsubstituted or is substituted by up to three identical or different substituents selected from the group consisting of halogen, alkyl having 1 or 2 carbon atoms and alkoxy having 1 or 2 carbon atoms;
or
A is the radical of a saturated or unsaturated lactam of the formula 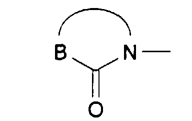
B is alkenylene or alkylene having 3, 4, 5 or 6 carbon atoms, which is unsubstituted or is substituted by up to three identical or different alkyl groups having 1, 2, 3 or 4 carbon atoms;
or
A is the radical of a bicyclic system of the formula 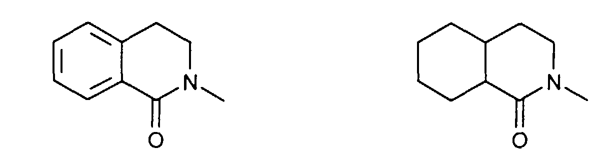 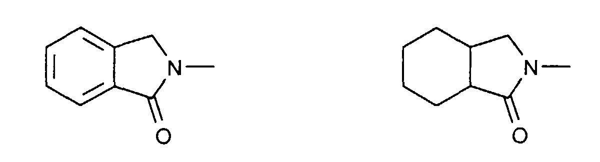
or its physiologically tolerable salts.

8. A compound of the formula I as claimed in at least one of claims 1, 2, 3 and 7, in which:
R(1) is hydrogen, alkyl having 1 or 2 carbon atoms or alkoxy having 1 or 2 carbon atoms;
R(2a), R(2b), R(2c), R(2d) and R(2e) are hydrogen;
R(3) is hydrogen, methyl or ethyl;
Q is (CH₂)ₙ;
n is 1 or 2;
Z is oxygen;
A is phenyl, which is unsubstituted or is substituted by up to three identical or different substituents selected from the group consisting of halogen, alkyl having 1 or 2 carbon atoms and alkoxy having 1 or 2 carbon atoms;
or
A is the radical of a saturated or unsaturated lactam of the formula 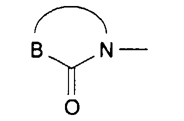
B is alkenylene or alkylene having 3, 4, 5 or 6 carbon atoms, which is unsubstituted or is substituted by up to three identical or different alkyl groups having 1, 2, 3 or 4 carbon atoms;
or its physiologically tolerable salts.

9. A compound of the formula I as claimed in at least one of claims 1, 2, 3, 7 and 8, in which:
R(1) is hydrogen, alkyl having 1 or 2 carbon atoms or alkoxy having 1 or 2 carbon atoms;
R(2a), R(2b), R(2c), R(2d) and R(2e) are hydrogen;
R(3) is hydrogen, methyl or ethyl;
Q is CH₂;
Z is oxygen;
A is phenyl, which is unsubstituted or is substituted by up to three identical or different substituents selected from the group consisting of halogen, alkyl having 1 or 2 carbon atoms and alkoxy having 1 or 2 carbon atoms;
or its physiologically tolerable salts.

10. A process for the preparation of compounds of the formula I as claimed in one or more of claims 1 to 9, which comprises reacting sulfamoylchromans of the formula II 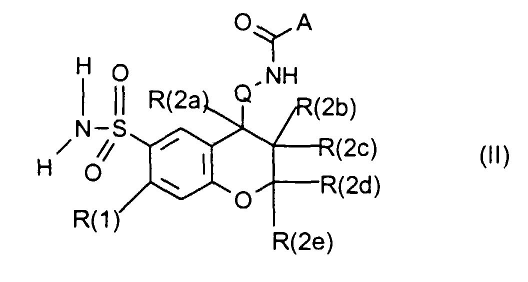 or their salts of the formula III 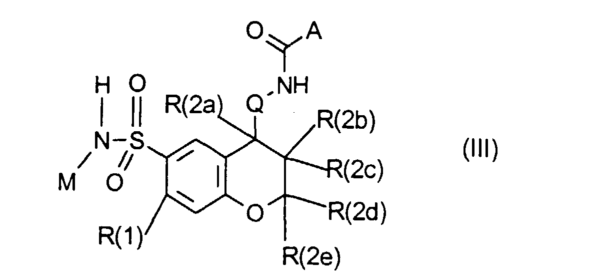 in which the radicals have the meanings indicated in claims 1 to 9 and the cation M is, for example, an alkali metal, alkaline earth metal, ammonium or tetraalkylammonium ion, with an R(3)-substituted isocyanate or isothiocyanate, an R(3)-substituted carbonic acid derivative or a trichloroacetamide which is R(3)-substituted on the nitrogen;
or, for the preparation of compounds of the formula I in which R(3) is hydrogen, reacting compounds of the formula II or III with a trialkylsilyliso(thio)cyanate or silicon tetraiso(thio)cyanate and cleaving the silicon-substituted chromanylsulfonyl(thio)ureas primarily formed;
or, for the preparation of compounds of the formula I in which Z is oxygen, desulfurizing compounds of the formula I in which Z is sulfur;
or, for the preparation of compounds of the formula I in which Z is oxygen, reacting compounds of the formula VII 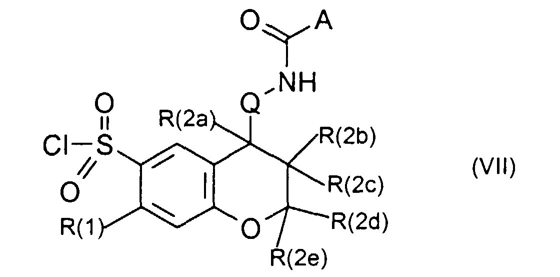 in which the radicals have the meanings indicated in claims 1 to 9, with an R(3)-substituted urea or bis(trialkyl)silylurea;
or reacting compounds of the formula VIII or of the formula IX 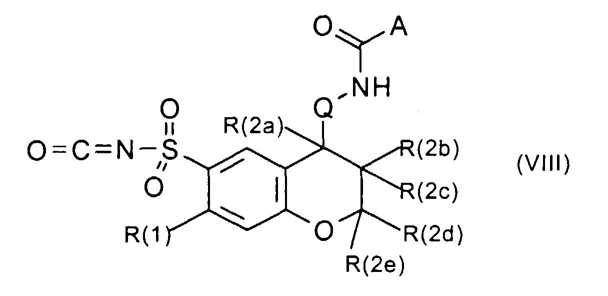 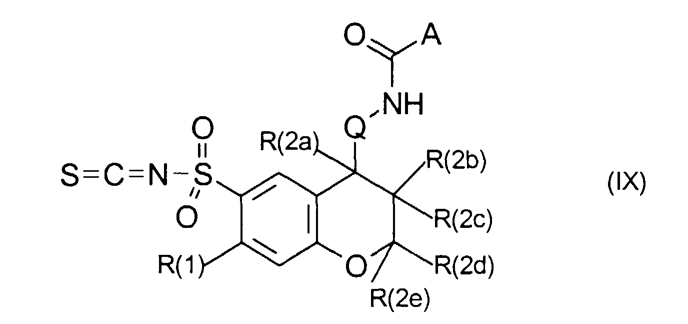 in which the radicals have the meanings indicated in claims 1 to 9, with an amine of the formula R(3)-NH₂.

11. The use of a compound of the formula I as claimed in one or more of claims 1 to 9 and/or of a physiologically tolerable salt thereof for the production of a medicament for the treatment of cardiac arrhythmias.

12. The use of a compound of the formula I as claimed in one or more of claims 1 to 9 and/or of a physiologically tolerable salt thereof for the production of a medicament for the prevention of sudden heart death.

13. The use of a compound of the formula I as claimed in one or more of claims 1 to 9 and/or of a physiologically tolerable salt thereof for the production of a medicament for the treatment of ischemic conditions of the heart.

14. The use of a compound of the formula I as claimed in one or more of claims 1 to 9 and/or of a physiologically tolerable salt thereof for the production of a scientific tool for the inhibition of ATP-sensitive potassium channels.

15. The use of a compound of the formula I as claimed in one or more of claims 1 to 9 and/or of a physiologically tolerable salt thereof for the production of a medicament for the treatment of weakened myocardial contraction force.

16. The use of a compound of the formula I as claimed in one or more of claims 1 to 9 and/or of a physiologically tolerable salt thereof for the production of a medicament for the improvement of cardiac function after heart transplantation.

17. A compound of the formula I as claimed in one or more of claims 1 to 9 and/or a physiologically tolerable salt thereof for use as a pharmaceutical.

18. A pharmaceutical which comprises an effective amount of at least one compound of the formula I as claimed in one or more of claims 1 to 9 and/or of a physiologically tolerable salt thereof.

## Revendications

1. Chromanylsulfonyl(thio)urées de formule I 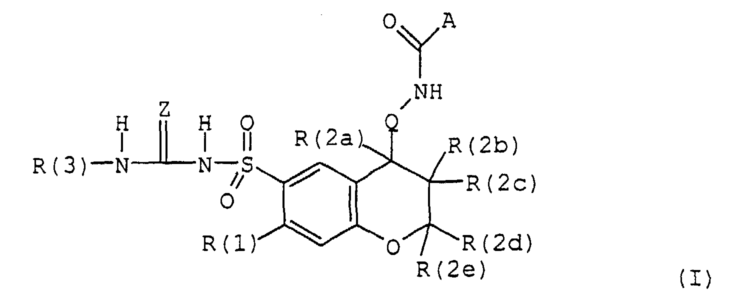 dans laquelle :
R(1) est l'hydrogène, un alkyle comportant de 1 à 4 atomes de carbone, un alcoxy comportant de 1 à 4 atomes de carbone, le fluor, le chlore, le brome, l'iode, CF₃, NH₂, un groupement NH-(alkyle comportant de 1 à 4 atomes de carbone), un groupement N(alkyle)₂ comportant de 1 à 4 atomes de carbone dans les groupments alkyles identiques ou différentes, ou un groupement S-alkyle comportant de 1 à 4 atomes de carbone ;
R(2a) est l'hydrogène ou un alkyle comportant 1 ou 2 atomes de carbone ;
R(2b) et R(2d) sont identiques ou différentes et sont l'hydrogène, un alkyle comportant 1 ou 2 atomes de carbone, un phényle non substitué, un phényle substitué, un benzyle non substitué ou un benzyle substitué au niveau du groupement phényle, les substituants étant présents au niveau du groupement phényle, jusqu'à trois substituants, identiques ou différents, choisis dans le groupe comprenant un halogène, un alkyle comportant 1 ou 2 atomes de carbone, un alcoxy comportant 1 ou 2 atomes de carbone ;
R(2c) et R(2e) sont identiques ou différents et sont l' hydrogène ou un alkyle comportant 1 ou 2 atomes de carbone ;
R(3) est l'hydrogène, un alkyle comportant 1, 2, 3 ou 4 atomes de carbone, un cycloalkyle comportant 3, 4, 5 ou 6 atomes de carbone au niveau du cycle, un groupement CH₂-cycloalkyle comportant 3, 4, 5 ou 6 atomes de carbone au niveau du cycle, ou CF₃ ;
Q est (CH₂)ₙ, avec n = 1 ou 2 ;
Z est le soufre ou l'oxygène ;
A est un phényle non substitué ou substitué par jusqu'à trois substituants identiques ou différents et choisis dans le groupe comprenant un halogène, un alkyle comportant 1 ou 2 atomes de carbone, un alcoxy comportant 1 ou 2 atomes de carbone ;
ou bien
A est un groupement lactame saturé ou insaturé de formule : 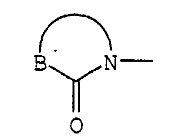 dans laquelle B est un alcénylène ou un alkylène comportant 3, 4, 5 ou 6 atomes de carbone, non substitué, ou substitué par jusqu'à trois groupes alkyles identiques ou différents comportant 1, 2, 3 ou 4 atomes de carbone ;
ou bien
A est un groupement correspondant à un système bicyclique d'une des formules suivantes 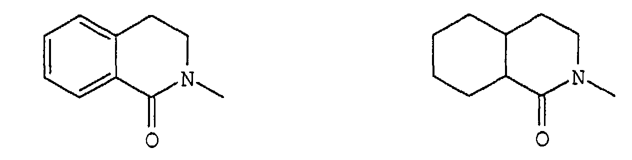 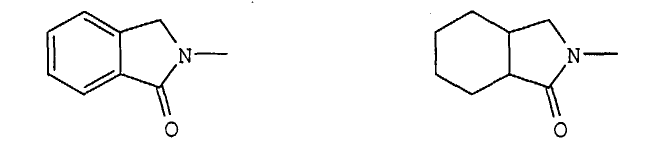
ainsi que leurs sels physiologiquement compatibles.

2. Composés de formule I selon la revendication 1, dans lesquels :
R(1) est l'hydrogène, un alkyle comportant 1 ou 2 atomes de carbone, un alcoxy comportant 1 à 4 atomes de carbone, le fluor, le chlore, le brome, l'iode ou CF₃ ;
R(2a), R(2b) et R(2d) sont identiques ou différents et sont l'hydrogène ou un alkyle comportant 1 ou 2 atomes de carbone ;
R(2c) et R(2e) sont des atomes d'hydrogène ;
R(3) est hydrogène ou un alkyle comportant 1, 2, 3 ou 4 atomes de carbone ;
Q est (CH₂)ₙ, avec n = 1 ou 2 ;
Z est le soufre ou l'oxygène ;
A est un phényle non substitué, ou substitué par jusqu'à trois substituants identiques ou différents choisis dans le groupe comprenant un halogène, un alkyle comportant 1 ou 2 atomes de carbone, un alcoxy comportant 1 ou 2 atomes de carbone ;
ou bien
A est un groupement lactame saturé ou insaturé de formule : 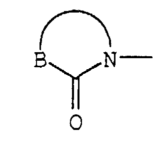 dans laquelle B est un alcénylène ou un alkylène comportant 3, 4, 5 ou 6 atomes de carbone, non substitué, ou substitué par jusqu'à trois groupes alkyles identiques ou différents comportant 1, 2, 3 ou 4 atomes de carbone ;
ou bien
A est un groupement correspondant à un système bicyclique d'une des formules suivantes : 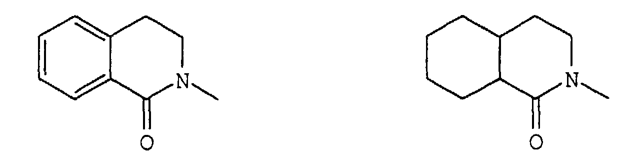 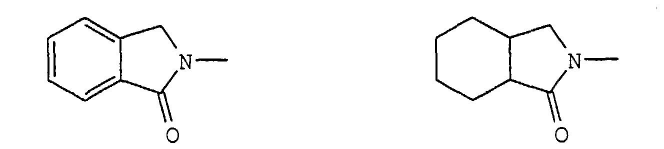
ainsi que leurs sels physiologiquement compatibles.

3. Composés de formule I selon la revendication 1 ou la revendication 2, dans lesquels :
R(1) est l'hydrogène, un alkyle comportant 1 ou 2 atomes de carbone, un alcoxy comportant 1 ou 2 atomes de carbone, le fluor, le chlore, le brome, l'iode ou CF₃ ;
R(2a), R(2b), R(2c), R(2d) et R(2e) sont des atomes d'hydrogène ;
R(3) est l'hydrogène ou un alkyle comportant 1, 2, 3 ou 4 atomes de carbone
Q est (CH₂)ₙ, avec n = 1 ou 2 ;
Z est le soufre ou l'oxygène ;
A est un phényle non substitué, ou substitué par jusqu'à trois substituants identiques ou différents choisis dans le groupe comprenant un halogène, un alkyle comportant 1 ou 2 atomes de carbone, un alcoxy comportant 1 ou 2 atomes de carbone ;
ou bien
A est un groupement lactame saturé ou insaturé de formule : 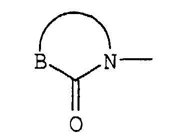 dans laquelle B est un alcénylène ou un alkylène comportant 3, 4, 5 ou 6 atomes de carbone, non substitué ou substitué par jusqu'à trois groupes alkyles identiques ou différents comportant 1, 2, 3 ou 4 atomes de carbone ;
ou bien
A est le groupement correspondant à un système bicyclique d'une des formules suivantes : 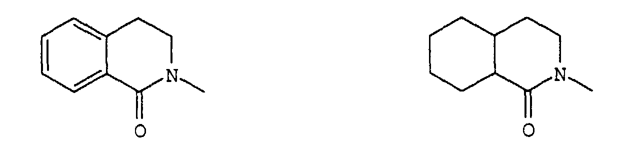 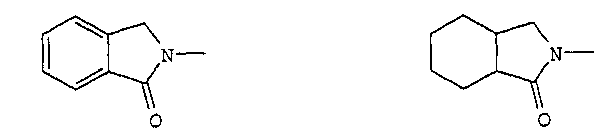
ainsi que leurs sels physiologiquement compatibles.

4. Composés de la formule I selon l'une au moins des revendications 1 à 3, dans lesquels :
R(1) est l'hydrogène, un alkyle comportant 1 ou 2 atomes de carbone ou un alcoxy comportant 1 ou 2 atomes de carbone ;
R(2a), R(2b), R(2c), R(2d) et R(2e) sont des atomes d'hydrogène ;
R(3) est l'hydrogène ou un alkyle avec 1, 2, 3 ou 4 atomes de carbone
Q est (CH₂)ₙ, avec n = 1 ou 2 ;
Z est le soufre ;
A est un phényle non substitué, ou substitué par jusqu'à trois substituants identiques ou différents choisis dans le groupe comprenant un halogène, un alkyle comportant 1 ou 2 atomes de carbone, un alcoxy comportant 1 ou 2 atomes de carbone ;
ou bien
A est un groupement lactame saturé ou insaturé de formule : 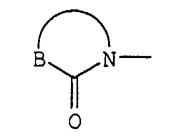 dans laquelle B est un alcénylène ou un alkylène comportant 3, 4, 5 ou 6 atomes de carbone, non substitué ou substitué par jusqu'à trois groupes alkyles identiques ou différents comportant 1, 2, 3 ou 4 atomes de carbone ;
ou bien
A est un groupement correspondant à un système bicyclique de l'une des formules suivantes : 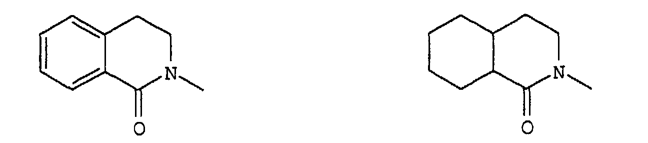 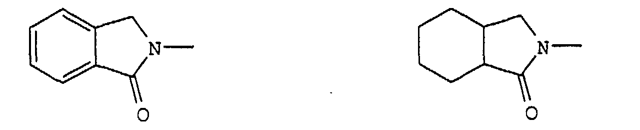
ainsi que leurs sels physiologiquement compatibles.

5. Composés de formule I selon l'une au moins des revendications 1 à 4, dans lesquels :
R(1) est l'hydrogène, un alkyle comportant 1 ou 2 atomes de carbone ou un alcoxy comportant 1 ou 2 atomes de carbone ;
R(2a), R(2b), R(2c), R(2d) et R(2e) sont des atomes d'hydrogène ;
R(3) est l'hydrogène, un méthyle ou un éthyle ;
Q est (CH₂)ₙ, avec n = 1 ou 2 ;
Z est le soufre ;
A est un phényle non substitué, ou substitué par jusqu'à trois substituants identiques ou différents choisis dans le groupe comprenant un halogène, un alkyle comportant 1 ou 2 atomes de carbone, un alcoxy comportant 1 ou 2 atomes de carbone ;
ou bien
A est un groupement lactame saturé ou insaturé de formule : 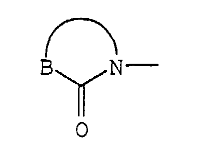 dans laquelle B est un alcénylène ou un alkylène comportant 3, 4, 5 ou 6 atomes de carbone, non substitué ou substitué par jusqu'à trois groupes alkyles identiques ou différents comportant 1, 2, 3 ou 4 atomes de carbone ;
ainsi que leurs sels physiologiquement compatibles.

6. Composés de formule I selon l'une au moins des revendications 1 à 5, dans lesquels :
R(1) est l'hydrogène, un alkyle comportant 1 ou 2 atomes de carbone ou un alcoxy comportant 1 ou 2 atomes de carbone ;
R(2a), R(2b), R(2c), R(2d) et R(2e) sont des atomes d'hydrogène ;
R(3) est l'hydrogène, un méthyle ou un éthyle ;
Q est CH₂ ;
Z est le soufre ;
A est un phényle non substitué, ou substitué par jusqu'à trois substituants identiques ou différents choisis dans le groupe comprenant un halogène, un alkyle comportant 1 ou 2 atomes de carbone, un alcoxy comportant 1 ou 2 atomes de carbone ;
ainsi que leurs sels physiologiquement compatibles.

7. Composés de formule I selon l'une au moins des revendications 1 à 3, dans lesquels :
R(1) est l'hydrogène, un alkyle comportant 1 ou 2 atomes de carbone ou un alcoxy comportant 1 ou 2 atomes de carbone ;
R(2a), R(2b), R(2c), R(2d) et R(2e) sont des atomes d'hydrogène ;
R(3) est l'hydrogène ou un alkyle comportant 1, 2, 3 ou 4 atomes de carbone ;
Q est (CH₂)ₙ, avec n = 1 ou 2 ;
Z est l'oxygène ;
A est un phényle non substitué, ou substitué par jusqu'à trois substituants identiques ou différents choisis dans le groupe comprenant un halogène, un alkyle comportant 1 ou 2 atomes de carbone, un alcoxy comportant 1 ou 2 atomes de carbone ;
ou bien
A est un groupement lactame saturé ou insaturé de formule : 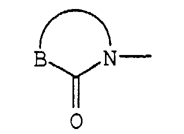 dans laquelle B est un alcénylène ou un alkylène comportant 3, 4, 5 ou 6 atomes de carbone, non substitué ou substitué, par jsqu'à trois groupes alkyles identiques ou différents comportant 1, 2, 3 ou 4 atomes de carbone ;
ou bien
A est un groupement correspondant à un système bicyclique de l'une des formules suivantes : 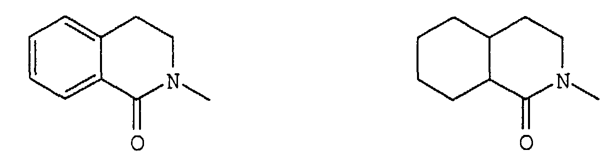 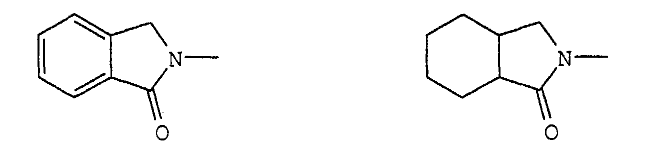
ainsi que leurs sels physiologiquement compatibles.

8. Composés de formule I selon l'une au moins des revendications 1, 2, 3 et 7, dans lesquels :
R(1) est l'hydrogène, un alkyle comportant 1 ou 2 atomes de carbone ou un alcoxy comportant 1 ou 2 atomes de carbone ;
R(2a), R(2b), R(2c), R(2d) et R(2e) sont des atomes d'hydrogène ;
R(3) est l'hydrogène, un méthyle ou un éthyle ;
Q est (CH₂)ₙ, avec n = 1 ou 2 ;
Z est l'oxygène ;
A est un phényle non substitué, ou substitué par jusqu'à trois substituants identiques ou différents choisis dans le groupe comprenant un halogène, un alkyle comportant 1 ou 2 atomes de carbone, un alcoxy comportant 1 ou 2 atomes de carbone ;
ou bien
A est un groupement lactame saturé ou insaturé de formule : 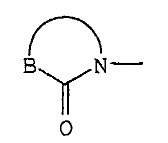 dans laquelle B est un alcénylène ou un alkylène comportant 3, 4, 5 ou 6 atomes de carbone, non substitué ou substitué par jusqu'à trois groupes alkyles identiques ou différents avec 1, 2, 3 ou 4 atomes de carbone ;
ainsi que leurs sels physiologiquement compatibles.

9. Composés de formule I selon l'une au moins des revendications 1, 2, 3, 7 et 8, dans lesquels :
R(1) est l'hydrogène, un alkyle comportant 1 ou 2 atomes de carbone ou un alcoxy comportant 1 ou 2 atomes de carbone ;
R(2a), R(2b), R(2c), R(2d) et R(2e) sont des atomes d'hydrogène ;
R(3) est l'hydrogène, un méthyle ou un éthyle ;
Q est CH₂ ;
Z est l'oxygène ;
A est un phényle non substitué, ou substitué par jusqu'à trois substituants identiques ou différents choisis dans le groupe comprenant un halogène, un alkyle comportant 1 ou 2 atomes de carbone, un alcoxy comportant 1 ou 2 atomes de carbone ;
ainsi que leurs sels physiologiquement compatibles.

10. Procédé de préparation de composés de formule I selon l'une ou plusieurs des revendications 1 à 9, caractérisé en ce que l'on fait réagir des sulfamoylchromanes de la formule II 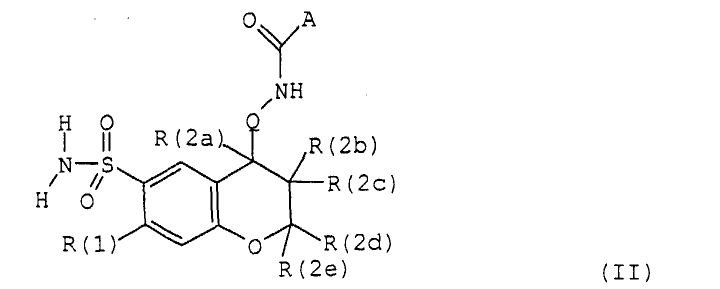 ou leurs sels de la formule III 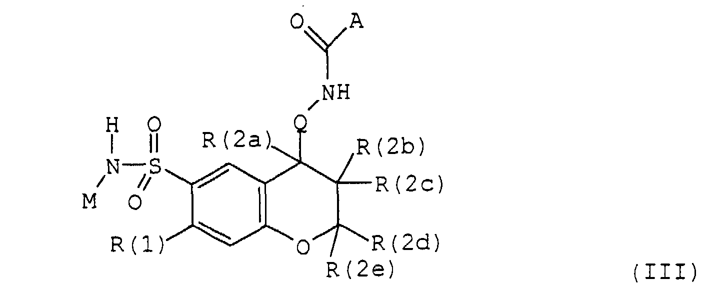 dans lesquelles les groupements ont les significations indiquées dans les revendications 1 à 9 et le cation M représente par exemple un ion de métal alcalin, de métal alcalino-terreux, un ion ammonium ou un ion tétraalkylammonium, avec un isocyanate ou isothiocyanate substitué par R(3), un dérivé d'acide carbonique substitué par R(3) ou un trichloracétamide substitué par R(3) au niveau de l'atome d'azote ;
ou bien, pour préparer des composés de formule I dans laquelle R(3) est l'hydrogène, on fait réagir des composés de formule II ou III avec un iso(thio)cyanate de trialkylsilyle ou un tétraiso(thio)cyanate de silicium et on dissocie les chromanylsulfonyl(thio)urées primaires substituées par le silicium ainsi obtenues ;
ou bien, pour préparer des composés de formule I dans laquelle Z est oxygène, on désoufre des composés de formule I dans laquelle Z est soufre ;
ou bien, pour préparer des composés de formule I dans laquelle Z est l'oxygène, on fait réagir des composés de formule VII 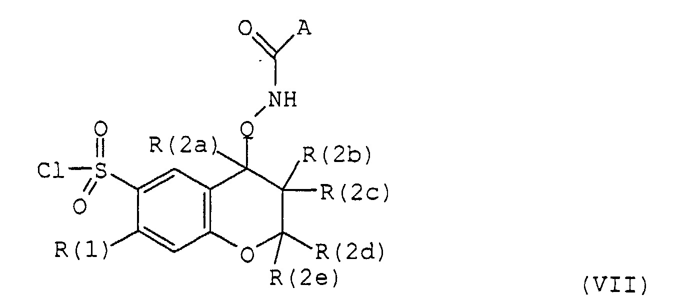 dans laquelle les groupements ont les significations indiquées dans les revendications 1 à 9, avec une urée substituée par R(3) ou une bis(trialkyl)silylurée ;
ou bien on fait réagir des composés de formule VIII ou de formule IX 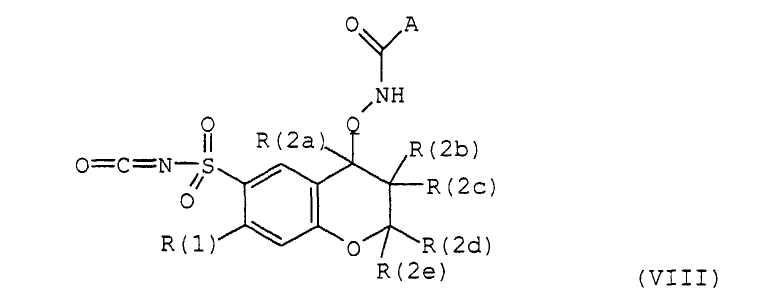 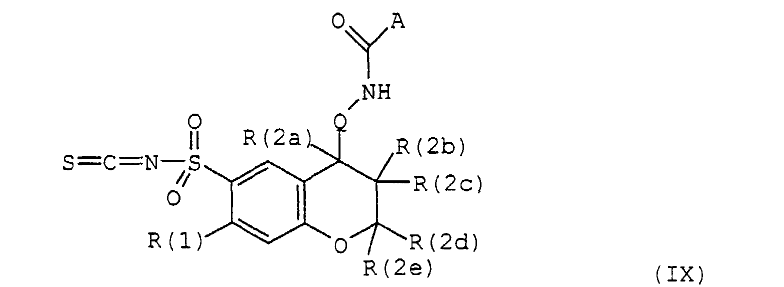 dans lesquelles les groupements ont les significations indiquées dans les revendications 1 à 9, avec une amine de la formule R(3)-NH₂.

11. Utilisation d'un composé de formule I selon l'une ou plusieurs des revendications 1 à 9 et/ou d'un sel physiologiquement compatible de celui-ci pour la préparation d'un médicament destiné au traitement des troubles du rythme cardiaque.

12. Utilisation d'un composé de formule I selon l'une ou plusieurs des revendications 1 à 9 et/ou d'un sel physiologiquement compatible de celui-ci pour la préparation d'un médicament destiné à prévenir la mort subite par arrêt cardiaque.

13. Utilisation d'un composé de la formule I selon l'une ou plusieurs des revendications 1 à 9 et/ou d'un sel physiologiquement compatible de celui-ci pour la préparation d'un médicament destiné au traitement des états ischémiques du coeur.

14. Utilisation d'un composé de la formule I selon l'une ou plusieurs des revendications 1 à 9 et/ou d'un sel physiologiquement compatible de celui-ci pour la préparation d'un outil scientifique pour l'inhibition des canaux potassiques sensibles à l'ATP.

15. Utilisation d'un composé de la formule I selon l'une ou plusieurs des revendications 1 à 9 et/ou d'un sel physiologiquement compatible de celui-ci pour la préparation d'un médicament destiné au traitement de l'insuffisance cardiaque.

16. Utilisation d'un composé de la formule I selon l'une ou plusieurs des revendications 1 à 9 et/ou d'un sel physiologiquement compatible de celui-ci pour la préparation d'un médicament destiné à améliorer le fonctionnement du coeur après une transplantation cardiaque.

17. Composé de la formule I selon l'une ou plusieurs des revendications 1 à 9 et/ou sel physiologiquement compatible de celui-ci destiné à être utilisé comme produit pharmaceutique.

18. Produit pharmaceutique, caractérisé en ce qu'il contient une quantité efficace d'au moins un composé de formule I selon l'une ou plusieurs des revendications 1 à 9 et/ou un sel physiologiquement compatible de celui-ci.
